(19) Europäisches Patentamt
European Patent Office
Office européen des brevets

(11) EP 1 391 508 A1

(12) EUROPEAN PATENT APPLICATION

(43) Date of publication:
25.02.2004 Bulletin 2004/09

(51) Int Cl.[7]: **C12N 15/00**, A01K 67/033, C07K 14/705, G01N 33/50, A61K 49/00

(21) Application number: **03017320.7**

(22) Date of filing: **31.07.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **09.08.2002 EP 02017918**

(71) Applicants:
- **F. HOFFMANN-LA ROCHE AG**
  **4070 Basel (CH)**
- **Givaudan SA**
  **1214 Vernier-Genève (CH)**

(72) Inventors:
- **Andres, Pedro J.**
  **4057 Basel (CH)**
- **Conte, Caroline**
  **68300 Saint-Louis (FR)**
- **Milani Muelhardt, Nicoletta**
  **79639 Grenzach-Wyhlen (DE)**
- **Nef, Patrick**
  **4125 Riehen (CH)**

# (54) TRANSGENIC NEMATODE EXPRESSING MAMMALIAN GPCRS USED FOR SCREENING ASSAY

(57) The present invention provides transgenic nematode strains expressing mammalian GPCRs useful for probing the function of a large number of mammalian GPCRs by linking simple nematode behaviors to the activation of mammalian GPCRs. The present invention also relates to methods for identifying substances that activate, antagonize or modulate mammalian GPCRs by determining the behavior of nematodes transgenic for the mammalian GPCRs towards the substances. The present invention particularly relates to nematodes transgenic for mammalian olfactory, taste and vomeronasal receptors and to methods using them.

EP 1 391 508 A1

SEQUENCE LISTING

<110> F. Hoffmann - La Roche AG

<120> Nematode screening assay

<130> Case 21334

<150> EP 02017918.0

<151> 2002-08-09

<160> 6

<170> PatentIn version 3.1

<210> 1

<211> 984

<212> DNA

<213> Rattus norvegicus

<300>

<301> Buck,L. and Axel,R.

<302> A novel multigene family may encode odorant receptors: a molecular basis for odor recognition

<303> Cell

<304> 65

<305> 1

<306> 175-187

<307> 1991

<308> M64386

<309> 1993-04-27

<313> (1)..(984)

<400> 1
atggagcgaa ggaaccacag tgggagagtg agtgaatttg tgttgctggg tttcccagct 60

cctgccccac tgcgagtact actatttttc ctttctcttc tggcctatgt gttggtgttg 120

actgaaaaca tgctcatcat tatagcaatt aggaaccacc caaccctcca caaacccatg 180

tattttttct tggctaatat gtcatttctg gagatttggt atgtcactgt tacgattcct 240

aagatgctcg ctggcttcat tggttccaag gagaaccatg gacagctgat ctcctttgag 300

gcatgcatga cacaactcta ctttttcctg ggcttgggtt gcacagagtg tgtccttctt 360

gctgtgatgg cctatgaccg ctatgtggct atctgtcatc cactccacta ccccgtcatt 420
gtcagtagcc ggctatgtgt gcagatggca gctggatcct gggctggagg ttttggtatc 480
tccatggtta aagttttcct tatttctcgc ctgtcttact gtggccccaa caccatcaac 540
cactttttct gtgatgtgtc tccattgctc aacctgtcat gcactgacat gtccacagca 600
gagcttacag actttgtcct ggccattttt attctgctgg gaccgctctc tgtcactggg 660
gcatcctaca tggccatcac aggtgctgtg atgcgcatcc cctcagctgc tggccgccat 720
aaagcctttt caacctgtgc ctcccacctc actgttgtga tcatcttcta tgcagccagt 780
attttcatct atgccaggcc taaggcactc tcagcttttg acaccaacaa gctggtctct 840
gtactctacg ctgtcattgt accgttgttc aatcccatca tctactgctt gcgcaaccaa 900
gatgtcaaaa gagcgctacg tcgcacgctg cacctggccc aggaccagga ggccaatacc 960
aacaaaggca gcaaaattgg ttag 984

<210> 2
<211> 21
<212> DNA
<213> Rattus norvegicus

<400> 2
gagagtgagt gaatttgtgt t 21

<210> 3
<211> 19
<212> DNA
<213> Rattus norvegicus

<400> 3
agagcggtcc cagcagaat 19

<210> 4
<211> 18
<212> DNA
<213> Caenorhabditis elegans

<400> 4
cagtggctca tgtcgagt 18

<210> 5
<211> 18
<212> DNA
<213> Caenorhabditis elegans

<400> 5

cgaccttctt tccatcat 18

<210> 6

<211> 900

<212> DNA

<213> Homo sapiens

<300>

<308> GenBank/AF227131

<309> 2000-03-18

<313> (1)..(900)

<400> 6

```
atgcttcggt tattctattt ctctgctatt attgcctcag ttattttaaa ttttgtagga    60
atcattatga atctgtttat tacagtggtc aattgcaaaa cttgggtcaa aagccataga   120
atctcctctt ctgataggat tctgttcagc ctgggcatca ccaggtttct tatgctggga   180
ctatttctgg tgaacaccat ctacttcgtc tcttcaaata cggaaaggtc agtctacctg   240
tctgcttttt ttgtgttgtg tttcatgttt ttggactcga gcagtgtctg gtttgtgacc   300
ttgctcaata tcttgtactg tgtgaagatt actaacttcc aacactcagt gtttctcctg   360
ctgaagcgga atatctcccc aaagatcccc aggctgctgc tggcctgtgt gctgatttct   420
gctttcacca cttgcctgta catcacgctt agccaggcat caccttttcc tgaacttgtg   480
actacgagaa ataacacatc atttaatatc agtgagggca tcttgtcttt agtggtttct   540
ttggtcttga gctcatctct ccagttcatc attaatgtga cttctgcttc cttgctaata   600
cactccttga ggagacatat acagaagatg cagaaaaatg ccactggttt ctggaatccc   660
cagacggaag ctcatgtagg tgctatgaag ctgatggtct atttcctcat cctctacatt   720
ccatattcag ttgctaccct ggtccagtat ctcccctttt atgcagggat ggatatgggg   780
accaaatcca tttgtctgat ttttgccacc ctttactctc caggacattc tgttctcatt   840
attatcacac atcctaaact gaaaacaaca gcaaagaaga ttctttgttt caaaaaatag   900
```

## Description

The present invention relates to methods for producing a transgenic nematode that expresses a mammalian G protein-coupled receptor (herein "GPCR") in its neurons that are linked to behavior. These transgenic nematode strains can be used in a variety of methods, including, but not limited to methods for (1) screening and identifying substances that activate mammalian GPCRs; (2) screening and identifying substances that antagonize mammalian GPCRs; (3) screening and identifying substances that modulate mammalian GPCR activity; and (4) evaluating the specificity and efficacy of substances on mammalian GPCR activation.

The superfamily of seven-transmembrane-domain G-protein-coupled receptors (GPCRs) is the largest and most diverse group of transmembrane proteins involved in signal transduction. Each of the about 1000 to 1500 family members found in vertebrates responds to stimuli as diverse as hormones, neurotransmitters, odorants and light, which selectively activate intracellular signaling events mediated by heterotrimeric G proteins. Because GPCRs are centrally positioned in the plasma membrane to initiate a cascade of cellular responses by diverse extracellular mediators, it is not surprising that modulation of GPCR function has been successful in the development of many marketed therapeutic agents. It has become clear that GPCRs for which a physiological activating ligand has not yet been identified (orphan GPCRs) might provide a path to discovering new cellular substances that are important in human physiology. The process of 'de-orphanizing', that means to identify the ligand, or a surrogate ligand, that can bind and activate the receptor, is a crucial step in understanding the function of a particular receptor (Howard AD, McAllister G, Feighner SD, Liu Q, Nargund RP, Van der Ploeg LH, Patchett AA (2001). Trends Pharmacol Sci:22(3):132-40).

Successful identification of ligands of orphan receptors involves three prerequisites; a) cell-surface expression of sufficient orphan GPCR, b) diverse collections of purified ligands or high-quality tissue extracts, and c) broad-based robust assays to detect receptor activation. Most assay systems used to date involve heterologous expression of receptors and determination of the stimulation of a second messenger system by measuring such parameters as calcium mobilization, cAMP, GTP-gamma S binding, inositol phosphate production, MAP kinase activation, etc., to identify activating substances. These assays involve either sophisticated machines that can detect receptor activation in transgenic mammalian cell lines (Sullivan, *et al., Methods Mol* Biol 114: 125-33 (1999)) or labor-intensive methods, such as microinjection of *Xenopus* oocytes followed by electrophysiological recording (Wagner, et al. (2000), Cell Physiol Biochem. 10(1-2): 1-12). In addition, such assays require prior knowledge of the major signal-transduction pathway used and involve direct mechanical detection of receptor function. Moreover, the heterologous expression used in these assaying methods might provide problems for certain receptors as the physiological molecular environment necessary for receptor expression and receptor signaling is not present. Therefore, some orphan GPCRs cannot be expressed and/or de-orphanized with the known heterologous expression and assay systems.

The superfamily of GPCRs can be devided into different families and subfamilies based on their sequence homology the members of which often share similar ligands. The olfactory receptor (ORs) family is the largest GPCR family. The ORs are expressed in the olfactory sensory neurons (OSNs) and are responsible for the recognition of odorants which allows detection and discrimination of environmental stimuli as well as communication with other individuals, influencing animal behaviour. It has been estimated that the human olfactory receptor gene family contains ~1000 genes with ~ 350 full-length, uninterrupted genes, the remaining being pseudogenes (Mombaerts, P. (2001) *Annu. Rev. Genomics Hum. Genet.* **2**, 493-510).

Many putative ORs have been identified in several organisms but for most of them their ligands are still unknown. Determining the ligand-receptor specificity of individual ORs is fundamental to understand how the olfactory system detects and discriminates among thousands of different odors, and to analyze the functional organization of the mammalian olfactory system.

Another family belonging to the GPCR superfamily are taste receptors which are responsible for part of the taste perception of an animal. The initial step in taste perception is the interaction of water-soluble molecules with receptors expressed at the surface of taste receptor cells. On binding taste molecules, the receptors trigger transduction cascades that activate synapses and thus cause activation of the nerve fibres (for review, see Lindemann, *Nature* **413**: 219-225 (2001). Mammals taste many compounds but are believed to distinguish between only five primary tastes: sweet, bitter, sour, salty and umami (the taste of monosodium glutamate). Electrophysiological studies suggest that sour and salty tastants modulate the function of taste receptor cells through membrane channels (Heck et al., *Science* **223**: 403-405 (1984); Kinnamon et al., *Proc. Natl. Acad. Sci. U S A* **85**: 7023-7027 (1988). Other primary tastes, sweet, bitter and umami are believed to be detected by G-protein-coupled receptor (GPCR) signalling pathways (Chaudhari and Roper, *Ann. N Y Acad. Sci.* **855**: 398-406 (1998); Wong et al. *Nature* **381**: 796-800 (1996). A number of taste receptor GPCRs called T1Rs, T2Rs and taste-mGluR4 have been recently identified and characterized in vertebrates. The taste cell-derived variant of mGluR4 receptor has been proposed to function as an umami taste receptor (Chaudhari et al., *Nat. Neurosci.* **3**: 113-119 (2000). The T1R family of taste GPCRs is thought to function in the perception of sweetness (Max et al., *Nat. Genet.* **28**: 58-63 (2001); Montmayeur et al., *Nat. Neurosci.* **4**: 492-498 (2001); Nelson et al., *Cell* **106**: 381-390 (2001); Sainz et al., *J. Neurochem.* **77**: 896-903 (2001)) and as amino acid sensors (Nelson et al., *Nature* **416**: 199-202 (2002). The T2R family of taste GPCRs is implicated in the perception of bitterness (Adler et al., *Cell* **100**: 693-702 (2000); Chandrashekar et al., *Cell* **100**: 703-711 (2000); Matsunami et al., *Nature* **404**: 601-604 (2000)).

The mechanism of bitterness perception is not well understood. A number of members of the T2R receptor family have been identified in human and rodents but very few of them are known to respond to bitter tastants (Adler et al., 2000; Chandrashekar et al., 2000; Matsunami et al., 2000). A single taste receptor cell seems to express a large number of T2Rs, suggesting that each cell may be capable of recognizing multiple bitter tastants (Adler et al., 2000). In contrast, functional imaging experiments show that only one out of five bitter compounds tested activated most bitter-responsive taste cells (Caicedo and Roper (2001), *Science* **291**: 1557-1560). These observations suggest that different bitter stimuli may activate, through the expressed members of the T2R family of taste GPCRs, different subpopulations of bitter taste cells.

Two gene families encoding more than 240 GPCRs that are expressed in the vomeronasal organ have been discovered in mouse and rat, the V1R family and V2R family (for review see Mombaerts, *Science* **286**: 707-711 (1999); Buck, *Cell* **100**: 611-618 (2000)). These receptor families are considered as putative pheromone receptors. In human, only one vomeronasal receptor has been identified: V1RL1 (Rodriguez et al. (2000), *Nature Genetics* **26**: 18-19).

Further progress in the elucidation of GPCR function, especially of the function of orphan GPCRs, whose signal transduction pathway is not known, has been limited by the absence of a reliable and efficient system for expressing and assaying cloned receptors. Especially for the study of odorant-OR activation and the identification of ligands for orphan ORs, a novel assaying system is required. Although the first mammalian putative odorant receptors were identified in the early nineties (Buck, L., and Axel, R. (1991) *Cell* **65**, 175-187), there are few reports showing functional expression of full-length ORs in vertebrate OSNs and heterologous systems (Raming, K., Krieger, J., Strotmann, J., Boekhoff, I., Kubick, S., Baumstark, C., and Breer, H. (1993) *Nature* **361**, 353-356; Krautwurst, D., Yau, K. -W., and Reed, R.R. (1998) *Cell* **95**, 917-926; Wetzel, CH., Oles, M., Wellerdieck, C., Kuczkowiak, M., Gisselmann, G., and Hatt, H. (1999) *J. Neurosci.* **19**, 7426-7433). The ability to functionally express ORs seems to be specific of mature OSNs and differentiated odora cells (Gimelbrant, A., Haley, S.L. and McClintock, T.S. (2001) *J. Biol. Chem.* **276**, 7285-7290; Murrell, J.R., and Hunter, D.D. (1999) *J. Neurosci.* **19**, 8260-8270). Odorant receptors are retained in the endoplasmic reticulum (ER) of non-neural cell types used for functional expression studies, like CHO, HEK-293, Xenopus melanophores and fibroblasts (McClintock, T.S., Landers, T.M., Gimelbrant, A.A., Fuller, L.Z., Jackson, B.A., Jayawickreme, C.K. and Lerner, M.R. (1997) *Mol. Brain Res.* **48**, 270-278; Gimelbrant, A., Stoss, T.D., Landers, T.M., and McClintock, T. (1999) *J. Neurochem.* **72**, 2301-2311). Therefore, a fast and reliable system to functionally express mammalian GPCRs, especially mammalian olfactory, taste and vomeronasal receptors was required.

*C. elegans* detects and discriminates volatile molecules through GPCRs and G protein-coupled signaling cascades which are similar to the sensory mechanisms in mammals (for review, see Mori, I. (1999) *Annu. Rev. Genet.* **33**, 399-422). In *C. elegans,* different types of sensory neurons, comprising AWA, AWB, AWC, ASH, ADL, ASE, ASJ, ASK, ASI, ASG, ADF and AFD neurons mediate different behaviors.

Techniques for performing *in vivo* assays using the nematode worm *C. elegans* as a model organism have been described in the art, e.g. in WO00/63427 and WO01/88532. As described, one of the main advantages of such assays involving the use of *C. elegans* is that they can be carried out in multi-well plate format. Additionally, assays involving the use of *C. elegans* can be carried out in an automated fashion, i. e. using suitable robotics. Furthermore, because assays involving the use of *C. elegans* may be automated, such assays are also ideally suited for screening of libraries of chemical compounds, in particular at medium to high throughput. Such automated screens may for instance be used in the discovery and/or development of new compounds (e. g. small molecules) for pharmaceutical use and for use as flavor or fragrance.

In contrast to the known *in vivo* assays with wild-type *C. elegans,* the present invention provides transgenic *C. elegans* strains expressing mammalian GPCRs useful for probing the function of a large number of GPCRs by linking simple *C. elegans* behaviors to the activation of mammalian GPCRs, therefore obviating the need for sophisticated instrumentation.

Also, unlike the most common used screening assays for receptor activation, the present invention does not require any prior information or has to rely on prior assumptions about the major signal-transduction pathway, e.g. the parameters to be measured (calcium, cAMP, *etc.).* Instead, the present invention uses simple biological behaviors in nematodes to assay receptor activation.

Therefore, the present invention provides a screening method which employs the sensory neurons of nematodes, preferably of *C. elegans,* as an expression system suitable for the expression of mammalian GPCRs, especially those of neuronal or sensory cells. Moreover, this nematode screening assay is feasible and may be upscaled to high-throughput format.

The present invention therefore provides methods for producing a transgenic nematode that expresses at least one mammalian G protein-coupled receptor (herein "GPCR") in its neurons that are linked to behavior. The invention also relates to transgenic nematodes expressing mammalian GPCRs in sensory neurons. Preferably, the invention relates to methods for producing transgenic nematodes expressing mammalian ordorant, taste or vomeronasal GPCRs and to the transgenic nematode strains produced by those methods. The preferred nematode in the present invention is *C. elegans.* The transgenic nematode strains can be used in a variety of methods, including, but not limited to methods for (1) screening and identifying substances that activate mammalian GPCRs; (2) screening and identifying substances that antagonize mammalian GPCRs; (3) screening and identifying substances that modulate mammalian GPCR activity; and (4) evaluating the specificity and efficacy of substances on mammalian GPCR activation. Preferred nematode behaviors assayed in these screening methods comprise water-soluble chemorepulsion, water-soluble chemoattraction, volatile chemorepulsion, volatile chemoattraction.

Generation of Transgenic Nematodes

In one aspect, the invention relates to a method for generating transgenic nematodes expressing at least one mammalian GPCR in a sensory neuron linked to behavior, wherein said method comprises the steps of (a) preparing at least one gene expression construct comprising a nucleotide sequence encoding a mammalian GPCR operably linked to a promotor specific for sensory neurons, (b) generating a transgenic nematode by introducing the said at least one gene expression construct into a nematode and (c) examining if the resulting nematode is a transgenic nematode expressing the at least one mammalian GPCR in a sensory neuron linked to behavior.

The present invention employs roundworms, nematodes, which are a large phylum that includes many of the helminths parasitic in man and a greater number of plant-parasitic and free-living soil and aquatic nonparasitic species. More specifically, the invention employs *Rhabditis.* Even more specifically, the present invention relates to the subgenus *Caenorhabditis,* comprising *Caenorhabditis briggsae, Caenorhabditis elegans, Caenorhabditis maupasi, Caenorhabditis remanei,* Caenorhabditis *sp.,* and *Caenorhabditis vulgaris*. Most preferred in the present invention is Caenorhabditis *elegans (C. elegans). C. elegans* is a simple soil nematode species that has been extensively described at the cellular and molecular level, and is a model organism for biological studies (See Riddle, *C. ELEGANS* II., Cold Spring Harbor Laboratory Press, Cold Spring Harbor, NY. (1997); The *C. elegans* Sequencing Consortium, Science **282**: 2012-2018. (1998); Sulston, WB (ed), The Nematode *Caenorhabditis Elegans,* Cold Spring Harbor Laboratory Press, Cold Spring Harbor, NY, pp. 123-155 (1988)). Different strains of *C. elegans* exist which can be used in the methods of the present invention. The adult hermaphrodite is composed of 959 cells, 302 of which are defined as neuronal cells. *C. elegans* can respond to a variety of substances in its environment by moving away from a point source, termed chemorepulsion, or moving towards a point source, termed chemoattraction. *C. elegans* also responds to a pheromone in the environment by taking an alternative developmental pathway, the dauer stage (see Riddle, 1997). Much of this chemosensory behavior is mediated by sensory organs termed the amphids, a pair of bilaterally symmetric structures located at the anterior of the animal. Each amphid contains the endings of 12 sensory neurons, eight of which are directly exposed to the environment through an opening in the cuticle of the animal. The cell bodies of the sensory neurons lie in the nerve ring of the animal, where they synapse with other neurons to pass on sensory information. The function of each amphid sensory neuron has been defined by laser ablation microsurgery and subsequent testing of the operated animal. Each type of sensory modulates a particular chemosensory behavior. For example, the AWA and AWC neurons mediate chemotaxis toward volatile compounds, including diacetyl, pyrazine, and thiazole; whereas the AWB neuron mediates chemorepulsive behavior toward volatile compounds. The ASI, ASG, ASE, ASK and ADF neurons mediate water-soluble chemoattraction, and the ASH and ADL neurons water-soluble chemorepulsion. The ASJ neurons are implicated in dauer formation. The AFD neurons mediate thermotaxic behavior, which includes thermoattraction and thermorepulsion. The observation that particular neurons are needed for different behaviors suggests that behavioral modalities are determined by the properties and synaptic connections of these neurons. While chemotactic behavior is determined at the neuronal level, the ability to discriminate among substances in the environment is determined by expression of particular receptors in the chemosensory neurons. The nematode detects and discriminates volatile chemicals through GPCRs and G-protein-coupled signaling cascades which are similar to the sensory mechanisms in mammals (Mori, I. (1999). *Annu. Rev. Genet.* **33**, 399-422). Particularly relevant for this view was the identification and characterization of the *odr-10* gene, which encodes the first G-protein coupled OR whose interacting ligand has been identified. ODR-10 is only expressed in AWA neurons and is necessary to detect the odorant diacetyl (Sengupta, P., Chou, J.H. and Bargmann, C.I. (1996) *Cell* **84**, 875-887). In AWA neurons, ODR-10 is coupled to a G-protein identified as a $G_i$-like alpha subunit (Roayaie, K., Crump, J.G., Sagasti, A., and Bargmann, C.I. (1998) *Neuron* **20**, 55-67). Unlike vertebrate olfactory receptors, coupled to $G_{olf}$ and believed to use cAMP as an intracellular messenger, at least in a majority of cases, to open cyclic nucleotide-gated (CNG) cation channels (Gibson, A.D., and Garbers, D.L. (2000) *Annu. Rev. Neurosci.* **23**, 417-439), the nematode chemosensory receptors seems to use cGMP in an alternative signaling pathway. Interestingly, ODR-10 has been expressed in mammalian cells and found to respond specifically to its volatile ligand diacetyl in $Ca^{2+}$ imaging assays (Wellerdieck, C., Oles, M., Pott, L., Korsching, S., Gisselmann, G., and Hatt, H. (1997) *Chem. Senses* **22**, 467-476; Zhang, Y., Chou, J.H., Bradley, J., Bargmann, C.I., and Zinn, K. (1997) *Proc. Natl. Acad. Sci. USA* **94**, 12162-12167), which indicate that ODR-10 also couples to the signaling pathway used by mammals. These results suggested a promiscuous interaction between *C. elegans* and mammalian proteins. Like mammalian ORs, also *C. elegans* chemoreceptors are inefficiently transported to the cellular surface in non-olfactory cells, suggesting the necessity of neuron specific cofactors for proper trafficking and membrane localization. Recently, it has been found that the membrane-associated protein ODR-4 and the subunit µl of the AP-1 clathrin adaptor UNC-101 function in the proper cellular localization of ORs in *C. elegans* chemosensory neurons (Dwyer, N.D., Troemel, E.R., Sengupta, P., and Bargmann, C.I. (1998) *Cell* **93**, 455-466; Dwyer, N.D., Adler, C.E., Crump, J.G., L'Etoile, N.D., and Bargmann, C.I. (2001) *Neuron* **31**, 277-287). Furthermore, ODR-4 improved the trafficking of rat U131 olfactory receptors in mammalian cells in culture (Gimelbrant, A., Haley, S.L. and McClintock, T.S. (2001) *J. Biol. Chem.* **276**, 7285-7290), suggesting a conservation in the trafficking mechanisms of ORs between *C. elegans* and mammals and further supporting a promiscuous interaction between *C. elegans* and mammalian proteins. Given that different neurons are involved in different chemotactic behaviors, the expression of a receptor in certain neurons likely determines the behavioral response to the substance recognized by the receptor. Researchers tested this hypothesis by misexpressing ODR-10 in the AWB neuron, which mediates volatile chemorepulsive behavior (Troemel, *et al.* (1997), *Cell* **91**: 161-169). This misexpression of ODR-10 led to a "reprogramming" of the chemotactic response, such that the animals showed chemorepulsive behavior to diacetyl, instead of chemoattraction. These observations lead to the assumption that ligand specificity is provided by the receptor itself, while the behavioral response is determined by which neuron the receptor is expressed in.

In one embodiment of the present invention, transgenic nematode strains are used as biosensors for substances that activate mammalian GPCRs or that block mammalian GPCR activation. This is accomplished by specific expression of mammalian GPCR(s) in the appropriate sensory neurons of nematode, allowing the organisms to sense and respond in a chemosensory fashion to substances that activate the mammalian GPCR(s). By expressing mammalian GPCR(s) in particular sensory neurons, the response of the animals to receptor activation will be manifested as one of several behaviors: chemorepulsion, chemoattraction, thermotaxis, including thermoattraction and thermorepulsion, or commitment to the dauer developmental pathway. Chemorepulsion may be from volatile compounds, mediated by the AWB sensory neuron of the preferred nematode, *C. elegans,* or from water-soluble compounds, mediated by its ASH and ADL sensory neurons. A mammalian GPCR can be expressed in the AWB neuron of *C. elegans* using the promoter region of the *str-1* gene operably linked to the receptor coding sequence (Troemel ER, et *al, Cell* **91** : 161-169 (1997)). A receptor can be expressed in the ASH and ADL neurons using the promoter region of the *nhr-79* gene, the *gpa-11* gene or the *ops-1* gene operably linked to the receptor coding sequence (Jansen G, et *al, Nature Genetics* **21**:414-419 (1999)). Chemoattraction can likewise be towards volatile compounds, mediated by the AWA and AWC neurons, or towards water-soluble compounds, mediated by the ASE, ADF, ASG, and ASI neurons. A receptor can be expressed in AWA neurons using the promoter region of the *odr-7* or *odr-10* gene operably linked to the receptor coding region. A receptor can be expressed in the ASE neuron using the promoter regions of the *flp-6, gcy-5, gcy-6, or gcy-7* genes operably linked to the receptor coding region (Vu S, *et al, PNAS* **91**:3384-3387 (1997)). Dauer formation is mediated by the ADF, ASI and ASJ neurons; a mammalian GPCR can be expressed in these neurons using the promoter region of the *gpa-11* gene operably linked to the receptor coding region. The promoter regions of the *daf-7, gpa-4* and *str-3* genes operably linked to the receptor coding region can also be used for expression in ASI neurons. A receptor can be expressed in the ASK neuron using the promoter regions of the *sra-7, sra-9, srg-2* and *srg-8* genes operably linked to the receptor coding region (Troemel ER, *et al* (1995), *Cell **83**:207-218).* Thermotaxis is mediated by the AFD neuron; a mammalian GPCR can be expressed in this neuron using the promoter of the *gcy-8* or the *nhr-38* gene operably linked to the receptor coding region. Once a mammalian GPCR is expressed in the appropriate neuron to elicit a desired behavior, these behaviors can then be used to, for example, identify the substances that act as ligands to activate the mammalian GPCR, identify substances that antagonize the mammalian GPCR activation, determine the mammalian GPCR that is activated by a particular substance, or determine the potency and efficacy of activating substances.

Therefore, in the methods of the present invention, the promotor specific for a sensory neuron is selected from the group comprising *daf-7,flp-6, gpa-2, gpa-4, gpa-11, gcy-5, gcy-6, gcy-7, gcy-8, nhr-38, nhr-79, odr-7, odr-10, ops-1, str-1, str-2, str-3, sra-7, sra-9, srg-2,* and *srg-8.*

The term "behavior," as used herein, refers to a behavior selected from the group comprising chemotaxis, thermotaxis, and dauer formation. More specifically, these behaviors encompass chemorepulsion from water-soluble substances, herein termed water-soluble chemorepulsion, chemoattraction towards water-soluble substances, herein termed water-soluble chemoattraction, chemorepulsion from volatile substances, herein termed volatile chemorepulsion, chemoattraction towards volatile substances, herein termed volatile chemoattraction, thermorepulsion, thermoattraction and dauer formation in response to pheromone sensation, herein termed dauer formation (Mori, I. ( 1999), *Annu. Rev. Genet., **33**:399-422).*

In one aspect of this method, the behavior is water-soluble chemorepulsion, and said sensory neurons are selected from the group comprising ASH and ADL neurons. In this method, the sensory neuron promotor is the promotor region of a gene selected from the group comprising *gpa-11, nhr-79* and *ops-1.* In another aspect, the present invention relates to the above-referenced method, wherein said behavior is water-soluble chemoattraction, and said sensory neurons are selected from the group comprising ASE, ADF, ASG, ASI and ASK neurons. In this method, the sensory neuron promotor is the promotor region of a gene selected from the group comprising *daf-7,flp-6, gcy-5, gcy-6, gcy-7, gpa-4, ops-1, sra-7, sra-9, srg-2, srg-8* and *str-3.* In yet another aspect of this method, the behavior is volatile chemorepulsion, and said sensory neurons are AWB neurons. In this embodiment of the invention, the sensory neuron promotor is the promotor region of *str-1.* In another aspect of this method, the behavior is volatile chemoattraction, and the sensory neurons are selected from the group comprising AWA and AWC neurons. In this embodiment, the sensory neuron promotor is the promotor region of a gene selected from the group comprising *str-2, gpa-2, odr-7* and *odr-10.* In another aspect of this method, the behavior is dauer formation, and said sensory neurons are selected from the group consisting of: ASI, ASG, and ADF neurons. In yet another aspect of this method, the behavior is selected from the group consisting of: thermoattraction and thermorepulsion, and said sensory neurons are AFD neurons. In this embodiment, the sensory neuron promoter is the promoter region of a gene selected from the group comprising *gcy-8* and *nhr-38.*

The gene expression construct used in this method can be either DNA or a plasmid. Gene expression constructs that direct expression of mammalian GPCRs will comprise the following different DNA segments: (1) a promoter segment; (2) a receptor-encoding segment; (3) a 3' segment and optionally (4) a signal peptide encoding segment or an enhancer segment. These constructs are then introduced into nematodes by methods well known in the art to generate a stable strain that expresses the receptors in the appropriate sensory neurons. The promoter DNA segment, importantly, confers the appropriate temporal and spatial expression to the mammalian GPCR. Genes that are expressed in particular sets of sensory neurons are known. Using recombinant DNA technique, the promoters or DNA segments that confer temporal/spatial specificity to these genes can be fused to a heterologous gene (Sambrook, *et al.,* 1. Plasmid Vectors. In Sambrook J, Fritsch EF, and Maniatis, T (eds), Molecular Cloning: A Laboratory Manual, Cold Spring Harbor Laboratory Press, Cold Spring Harbor, New York. (1989); F.M. Ausubel, R. Brent, R.E. Kingston, D.D. Moore, J.G. Seidman, J.A. Smith, K. Struhl (eds). Current Protocols in Molecular Biology. Current Protocols 1987-1994; John Wiley & Sons, Inc. 1994-1998). For example, reporter gene expression in the ADL and ASH neurons, both of which mediate avoidance behavior when exposed to water-soluble substances, can be driven by the *gpa-11* promoter (Jansen, *et al., Nature Genetics* **21**: 414-419 (1999)). Any such promoter or DNA regulatory element is suitable, provided it directs gene expression specifically, and solely, in sensory neurons that mediate a particular behavior. The promoter segment should include all necessary DNA to provide transcriptional initiation, 5' mRNA processing, and translational initiation at a methionine start codon. In order to bind exogenously applied substances, mammalian GPCRs are, preferably, localized to the cell surface membrane of the expressing cells. Proteins can be directed to the cell surface by virtue of a signal peptide sequence that is found at the amino-terminal portion of the protein (Heijne, *Nucleic Acids Research* ***14***: 4683-4690 (1986)). This signal peptide sequence can be introduced in one of two ways. First, the DNA segment encoding the receptor itself may already include a functional signal peptide sequence. Thus, directing the mammalian GPCR to the cell surface, where it can respond to extracellular molecules, can be accomplished by maintaining the peptide signal sequence of an inserted receptor protein coding sequence. Alternatively, a separate DNA segment that encodes a peptide signal sequence can be introduced between the promoter and receptor-encoding DNA. Ideally, the receptor-encoding segment of DNA will include the nucleotides necessary to encode the complete mammalian GPCR peptide, from the start methionine codon to the translational termination, or stop, codon. The DNA segment should be operably linked to the promoter DNA segment such that translational initiation will begin at the start methionine codon of the receptor- encoding DNA segment. As used herein, the term, "operably linked" refers to a DNA sequence and a regulatory sequence(s) that are connected in such a way as to permit gene expression when the appropriate molecules, e.g., transcriptional activator proteins, are bound to the regulatory sequence(s). A nucleic acid is "operably linked" when it is placed into a functional relationship with another nucleic acid sequence. For example, DNA for a presequence or a secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However, not all regulatory sequences have to be contiguous, e.g. enhancers. Linking is accomplished by ligation at convenient restriction sites. If such sites do not exist, then synthetic oligonucleotide adaptors or linkers are used in accordance with conventional practice. A 3' DNA segment is necessary to provide the appropriate transcriptional termination and 3' mRNA processing signals. Although the 3' regions of many genes may be sufficient for this purpose, the standard DNA segment used for expression in *C. elegans* is composed of a 3' portion of the UNC-54 gene (Fire A., *et al.,* Gene **93**: 189-198 (1990)). See Figure 1, which depicts DNA constructs that are capable of driving expression of a non-nematode cell surface receptor in the AWA and AWB neurons of **C.** *elegans* mediate volatile chemotaxis. Plasmid constructs can be introduced into **C.** *elegans* using described transformation methods to generate animals transgenic for the plasmid constructs (Mello, *et al., EMBO J.* **10**: 3959-3970 (1991)). "Transformation" means introducing DNA into an organism so that the DNA is replicable, either as an extrachromosomal element or by chromosomal integration. Depending on the host cell used, transformation is done using standard techniques appropriate to such cells. The calcium treatment employing calcium chloride, as described by Cohen, S. N., *Proc. Natl. Acad. Sci. (USA),* **69**: 2110 (1972) and Mandel, *et al., J. Mol. Biol.* **53**: 154 (1970), is generally used for prokaryotes or other cells that contain substantial cell-wall barriers. For mammalian cells without such cell walls, the calcium phosphate precipitation method of Graham, *et al., Virology* **52**: 456-457 (1978) is preferred. General aspects of mammalian cell host system transformations have been described in U.S. Pat. No. 4,399,216. However, other methods for introducing DNA into cells such as by nuclear injection, electroporation or by protoplast fusion may also be used. Transformations into *C. elegans* can be carried out by microinjection, as described in Mello CC, *et al, EMBO J.* **10**:3959-3970 (1991), or by microparticle bombardment as described in Wilm T, *et al.,* Gene **229**:31-35 (1999) and Praitis *et al., Genetics* **157**, 1217-26 (2001). Preferably, the plasmid used for this method is a linear plasmid. An important aspect of transformation in *C. elegans* is that plasmid constructs are easily co-transformed. Therefore, transgenic strains of *C. elegans* can be generated that express more than one mammalian GPCR. In addition, dominant co-transformation markers can be used to score for transgenic *C. elegans.* In a particularly preferred embodiment of the invention, a co-transformation marker that does not affect the ability of the animals to exhibit chemotactic behavior is used. This co-transformation marker can be, for instance, a DNA fragment that complements a mutant phenotype. For example, mutations in the *C. elegans unc-119* gene cause a severe movement defect. Introduction of a segment of DNA containing the wildtype copy of the *unc-119* gene will complement the movement defect (Maduro, *Genetics* **141**: 977-88 (1995)), causing the transgenic animals to be wildtype in movement. Cotransforming UNC-119 animals with the *unc-119* wildtype DNA and the sensory neuron expression plasmids will allow the presence of the transgene to be scored by rescue of the *unc-119* mutant phenotype. Alternatively, a reporter gene can be used that can be scored in a living animal, but does not affect the movement of the animal. For example, green fluorescent protein (herein referred to as "GFP") is a widely used reporter molecule in living systems (Ellenberg, *Trends Cell Biol.* **9(2)**: 52-56 (1999); Chalfie, *et al., Science* **263(5148)**: 802-05 (1994)). Co-injection of a GFP-expressing DNA construct with the sensory neuron expression plasmids would allow the scoring for the presence of the transgene by examination of animals for GFP fluorescence.

Therefore, in another aspect, this method of producing transgenic nematodes further comprises the step of introducing a gene expression construct that expresses a reporter gene with a nematode promotor. Preferably, this reporter gene encodes a fluorescent protein, such as a green fluorescent protein (GFP). Expression and activation of a particular mammalian GPCR may not be sufficient to produce a behavioral response from a transgenic nematode. This may happen if the receptor requires accessory proteins to be expressed at the cell surface or to activate downstream signaling pathways. For example, mammalian GPCRs activate downstream pathways through the action of heterotrimeric G proteins. Although the preferred nematode, *C. elegans* contains 20 G$\alpha$, 2 G$\beta$ and 2G$\gamma$ proteins, efficient activation of signaling pathways and subsequent behavioral response of the animal may require the coexpression of accessory proteins, such as G proteins. In a preferred embodiment of the invention, such accessory proteins are mammalian G proteins. Additionally, other accessory proteins like chaperones might be necessary for the efficient transport to and expression in the cell membrane. This has been shown for sensory GPCRs, e.g. olfactory receptor (Sheng M., Kim E. (2000)), J. Cell Sci. 113, 1851-61). This can be accomplished using the same gene expression constructs used to express the mammalian GPCR, replacing the receptor-encoding DNA segment with a DNA segment encoding the accessory protein. The constructs encoding the receptor and the accessory protein can then be cotransformed into the nematode.

The mammalian GPCRs of the present invention include all protein molecules which have been identified as GPCRs by their sequence homology and their seven-transmembrane-domain structure. Preferred are mammalian olfactory, mammalian taste and mammalian vomeronasal GPCRs. More preferred are olfactory GPCRs, taste GPCRs belonging to the T2R, T1R and umami families, and vomeronasal GPCRs belonging to the V1R and V2R families. Most preferred are olfactory GPCRs, taste GPCRs belonging to the T2R, T1R families and to taste-mGluR receptors, and vomeronasal GPCRs belonging to the V1R and V2R families (e.g. human V1RL1 receptor) from rat and human.

This invention further provides a method for generating a transgenic nematode expressing at least one mammalian GPCR in a sensory neuron linked to behavior, wherein said method comprises the following steps: (a) preparing at least one gene expression construct comprising a nucleotide sequence encoding a mammalian GPCR operably linked to a promotor specific for sensory neurons and a further gene expression construct comprising a nucleotide sequence encoding an accessory protein operably linked to a promotor specific for sensory neurons, (b) generating a transgenic nematode by introducing the at least one gene expression construct encoding a mammalian GPCR and the gene expression construct encoding an accessory protein into nematode and (c) examining if the resulting nematode is a transgenic nematode co-expressing the at least one mammalian GPCR and the accessory protein.

In a further embodiment of this method it is determined if the transgenic nematode exhibits a behavioral phenotype. In very general terms, phenotype is the observable character of a cell or an organism. Therefore, in the transgenic nematode of the invention (expressing mammalian olfactory or taste or vomeronasal receptors), the observed phenotypes are the different behavioral responses toward volatile or soluble molecules in relation to the behavioral responses observed toward those molecules in wild type nematode (which do not express mammalian olfactory or taste or vomeronasal receptors).

Moreover, a transgenic nematode expressing a mammalian GPCR in a sensory neuron linked to behavior is provided by the present invention.

A further aspect relates to a transgenic nematode co-expressing a mammalian GPCR and an accessory protein in a sensory neuron linked to behavior.

Preferred are transgenic nematodes expressing a mammalian taste, olfactory or vomeronasal GPCR.

Another embodiment of this invention encompasses a transgenic nematode produced by any of the above-described methods.

Assaying Behavior in Transgenic Nematodes

Test populations of transgenic nematodes that express mammalian GPCRs in their sensory neurons will be able to sense and react to molecules or substances that modify receptor activity. Depending on the sensory neuron(s) that the mammalian GPCR(s) are expressed in, the reaction may be movement toward, or movement away from, a source of the substance, thermotaxis behavior, or commitment to the dauer pathway. A large variety of assays can be configured based on these behaviors.

Therefore, in a further embodiment, this invention provides a method for identifying at least one ligand of at least one mammalian GPCR, said method comprising the steps of (a) providing at least one nematode that expresses at least one mammalian GPCR in sensory neurons that are linked to behavior; (b) contacting said at least one nematode with at least one test substance; and (c) detecting modulation of behavior of said at least one nematode in response to said at least one test substance.

In another embodiment, the invention provides a method for identifying at least one test substance that is an agonist of a mammalian GPCR, said method comprising the steps of (a) providing at least one nematode that expresses at least one mammalian GPCR in sensory neurons that are linked to behavior; (b) contacting said at least one transgenic nematode with the at least one test substance; (c) determining whether said at least one test substance causes an activation of the said behavior in said at least one transgenic nematode; and (d) identifying said at least one test substance that causes an activation of said behavior in said at least one transgenic nematode as an agonist of said mammalian GPCR.

In yet another particularly preferred embodiment of the invention, screening methods can be used to identify an antagonizing substance that blocks the effect of a substance on mammalian GPCR. For example, if a ligand-receptor pair is already known, the present invention can be used to screen for or evaluate the ability of substances to antagonize receptor activation by the ligand. The chemosensory response of animals to the ligand in the presence and absence of substances can be assayed. If a substance antagonizes receptor activation by the ligand, then the chemotactic response of the animals to the ligand will be abolished in the presence of antagonist. If the test substance agonizes ligand activation of the receptor, then the chemotactic response should be enhanced in the presence of the substance.

Therefore, a method is provided for identifying at least one test substance that is an antagonist of a mammalian GPCR, said method comprising the steps of (a) providing at least one nematode that expresses at least one mammalian GPCR in sensory neurons that are linked to behavior, wherein said at least one mammalian GPCR has a known agonist which activates said behavior; (b) contacting said at least one transgenic nematode with the at least one test substance in the presence of the known agonist; (c) determining whether said at least one test substance causes a suppression of the said behavior in said at least one transgenic nematode; and (d) identifying said at least one test substance that causes a suppression of said behavior in said at least one transgenic nematode as an antagonist of said mammalian GPCR.

Furthermore, a method for identifying at least one test substance that is a modulator of a mammalian GPCR is provided, said method comprising the steps of (a) providing at least one nematode that expresses at least one mammalian GPCR in sensory neurons that are linked to behavior, wherein said at least one mammalian GPCR has a known agonist or antagonist which activates or inhibits said behavior; (b) contacting said at least one transgenic nematode with the at least one test substance in the presence of the known agonist or antagonist; (c) determining whether said at least one test substance causes a modulation of the said behavior in said at least one transgenic nematode; and (d) identifying said at least one test substance that causes a modulation of said behavior in said at least one transgenic nematode as an modulator of said mammalian GPCR.

The term "ligand" as used herein is defined as a substance that binds to a receptor. The term "ligand" comprises physiological, endogenous ligands and artificial, exogenous ligands. Ligands that bind to a receptor and elicit a positive response, e.g. activate the receptor, are defined as agonists. Ligands with effects opposite to those of an agonist act as antagonists. Ligands that modulate the effects of agonists or antagonists on receptors are called modulators. A modulator also comprises an allosteric enhancer and an allosteric inhibitor.

In a preferred embodiment of the invention, substances are presented to test populations of transgenic nematodes, preferably, transgenic *C. elegans* in such a way that allows chemosensory behavior. In such chemotaxis assays, preferably, the animals are placed on a substrate that allows free movement, such as a solid or a semi-solid substrate. For example, a typical culture medium for *C. elegans* is composed of a buffered solidified agar substrate placed in a container such as a petri plate. The solidified substrate may additionally be humified with buffer at the surface. In order to observe chemotactic behavior, it is preferable that a test substance is present in at least one concentration in a defined zone of the substrate. A number of standard chemotactic assays have been described, including assays that use point sources, gradients, and simple two concentration presentation of test substances. All of these assays have the following two characteristics: (1) a substrate medium allows the animals to locomote efficiently between, and show a preference for, different zones of the substrate; and (2) the presence of a test substance at one concentration or more in spatially distinct zones of the substrate medium. Test substances can be presented in a dispersed format, or a point source format. Examples for chemotactic assays with soluble substances are described in Tajima et al., Bioscience and Bioengineering 91: 322-324 (2001); Wicks et al., Developmental Biology 221: 295-307 (2000), and Jansen et al., EMBO J. 21: 986-994 (2002). An example of a dispersed format presentation method is the water-soluble chemorepulsive assay that is performed in divided petri dishes. In this format, a test substance is added to the agar poured in one half of a divided petri dish. In the other half of the petri dish, agar without the substance is poured. A small drop of isoamyl alcohol, which is a strong diffusable chemoattractant to *C. elegans,* is placed on the agar half that contains the test substance and a population of test animals is placed on the opposite half. Other known chemoattractants can be used, such as biotin, lysine, histidine, cysteine, and serotonin in place of the isoamyl alcohol. After a suitable time period, typically 90 minutes, the dispersion of animals on each half of the plate is quantitated by one of the methods discussed above. The fraction of animals that remain on the non-substance containing agar half of the petri plate is a reflection of how well the test substance can "repel" the animals. In assays with strong chemorepellants, such as copper sulfate, sodium dodecyl sulfate (SDS), D-tryptophan, heptanol, octanol, nonanol, nonanone, benzaldehyde, 2, 4, 5trimethylthiazole, and ethyl heptanoate, more than 90% of the animals typically remain on the half of the agar plate without the chemorepellant.

In a particularly preferred embodiment, the present invention provides the aforementioned methods wherein step (b) comprises placing a medium in a container; placing the at least one test substance on said medium; adding said at least one nematode to said container; and detecting, after a suitable time period, the behavioral response of said at least one nematode over the surface of said medium.

In a further embodiment of the methods of the present invention, the test substance is a water-soluble chemotactic substance and is placed on the medium in a defined zone of the container.

In yet another embodiment of the methods of the present invention, the test substance is a volatile chemotactic substance and is placed on the medium at one end of the container.

The behavior in the methods of the present invention may be a chemorepulsive behavior which comprises volatile and water-soluble chemorepulsive behavior and the test substance may be a chemorepulsive substance; or the behavior may be a chemoattractive behavior which comprises volatile chemoattractive behavior and water-soluble chemoattractive behavior and the test substance may be a chemoattractant substance. Point sources of substances could also be used to test chemorepulsion and/or chemoattraction. The square plate assay is an example of a point source assay used to measure both chemoattraction and chemorepulsion (See Troemel, *et al., supra).* Two spots of test substance are placed on the surface at one end of a square agar plate. Two spots of a control substance are placed at the other end, and a test population of *C. elegans* is placed in the center of the plate. After a suitable time period, the distribution of animals on the plate is quantitated. If the test substance is chemoattractive, the animals will tend to move toward that end of the plate. If the test substance is chemorepulsive, the animals will move away from that end of the plate. Thereby, zones of chemoattraction or chemorepulsion can be defined depending on the distance from the source of the test substance. Although a square plate facilitates quantitation of the animals, the shape is not important for behavior. Point sources of substances could be used with round plates, for example. In addition, presentation of substances as point sources can be used to assay large numbers of test substances by creating an array of spots of at least one test substance on a substrate medium. A number of different test substances can be placed in an array of spots on a plate. A test population of transgenic nematodes generated, as described above, can then be introduced and assayed to determine their chemotactic preference for the test substances. Spots of test substances that the nematodes preferentially avoid, or are attracted to, would then be candidates for substances that modify the activity of mammalian GPCRs that are expressed in the sensory neurons of the test population of nematodes. In a particularly preferred embodiment of the present invention, the test substances can easily diffuse in the substrate medium. Presenting test substances as an array of point sources confers the advantage of being able to rapidly assay a large number of different point sources of substance for their effect on chemotactic behavior.

In yet another aspect, the screening methods of the invention, wherein the test substance is a volatile test substance, further comprise the following step: (f) generating a chemotactic index, wherein said chemotactic index (C. I.) is calculated using the formula

$$C.I.= ((1+2) - (5+6)) / N$$

where 1, 2, 5, and 6 are the number of worms in the corresponding zones of the container, and N is the total number of worms in all six zones of the container, zone 1 contains the volatile test substance in a solvent and zone 6 contains the solvent as a control (Fig. 3A). A chemotaxis index of +1.0 indicates total attraction, an index of-1.0 indicates total repulsion.

In yet another aspect, the screening methods of the invention, wherein the test substance is a water-soluble test substance, further comprise the following step: (f) generating a chemotactic index, wherein said chemotactic index (C.I.) is calculated using the formula

$$C.I. = (1-2) / N$$

where 1, 2 are the number of worms in the corresponding zones of the plate, and N is the total number of worms in both zones of the plate, zone 1 contains the dissolved test substance and zone 2 contains no test substance. A chemotaxis index of+1.0 indicates total attraction, an index of-1.0 indicates total repulsion (Fig. 3B).

In another aspect of the methods of the present invention, said sensory neurons are selected from the group comprising: ASE, ADF, ASG, ASK and ASI neurons for water-soluble chemoattraction; AWA and AWC neurons for volatile chemoattraction; ASH and ADL neurons for water-soluble chemorepulsion; AWB neurons for volatile chemorepulsion; ASJ neurons for dauer formation in response to pheromones; as well as AFD neurons for thermotaxis. Moreover, in the methods of the present invention, the promotor specific for a sensory neuron is selected from the group comprising daf-7, *flp-6, gpa-2, gpa-4, gpa-11, gcy-5, gcy-6, gcy-7, gcy-8, nhr-38, nhr-79, odr-7, odr-10, ops-1, str-1, str-2, str-3, sra-7, sra-9, srg-2,* and *srg-8.*

In yet another aspect of this screening method, said detecting step (e) is carried out by direct detection with a suitable optical instrument. Said detecting step (e) can be carried out by assisted detection of the optical density over the surface of said medium. Alternatively, said assisted detection can be mechanical detection.

The term, "chemoattractant substance", as used herein, refers to both known chemoattractants, such as isoamyl alcohol, biotin, lysine, histidine, cysteine, and serotonin (Bargmann CI., *Chemotaxis and Thermotaxis,* In Riddle, Blumenthal, Meyer, and Priess (eds.), C. *ELEGANS* II, Cold Spring Harbor Laboratory Press, Cold Spring Harbor, NY (1997)), as well as substances that are discovered to elicit chemoattractive behavior from transgenic nematodes of the present invention. The term, "chemorepellant substance", as used herein, refers to both known chemorepellants, such as copper sulfate, sodium dodecyl sulfate (SDS), D-tryptophan, heptanol, octanol, nonanol, nonanone, benzaldehyde, 2, 4, 5-trimethylthiazole, and ethyl heptanoate as well as substances that are identified by eliciting chemorepulsive behavior from transgenic nematodes of the present invention.

The term, "test population," as it is used herein, refers to a group of nematodes from the same strain that each express the same mammalian GPCR(s).

The term, "test substance," as used herein, refers to an organic or inorganic molecule, a homogenous mixture of such molecules, biological extracts, or biological fractions. A "test substance" could be, for example, a viral plaque or a natural peptide. A "test substance" could be, for example, a known chemoattractant or chemorepulsant compound or a molecule that is discovered to elicit chemosensory behavior from transgenic nematodes of the present invention. "Test substances" could be derived from chemical libraries.

Yet another particularly preferred embodiment of the invention is a chemotaxis assay that places a test population of nematodes on a biomolecular separation matrix. Such a biomolecular separation matrix could be in agarose or polyacrylamide, and the animals could then be placed on the matrix. By monitoring the behavior of the animals, any preference for certain areas of the matrix could be determined. For example, a tissue that might contain a ligand for a mammalian GPCR could be fractionated by molecular mass in a matrix-based separation system. The chemotactic behavior of animals that express the mammalian GPCR in their sensory neurons could then indicate what region of the matrix contained the ligand of the mammalian GPCR, and therefore, what mass the ligand was. There are a large variety of separation techniques for biological samples, including those for small peptides, proteins, DNA, and other biological components. Many of these techniques use a matrix substrate that is amenable to a chemotaxis assay using test populations of nematodes. For example, nucleic acids and proteins can be separated in a simple agarose substrate (Sambrook, *et al.,* 6. Gel Electrophoresis of DNA, In Sambrook J, Fritsch EF, and Maniatis, T (eds), Molecular Cloning: A laboratory Manual, Cold Spring Harbor Laboratory Press, Cold Spring Harbor, New York. (1989); Sambrook, *et al,* 18 Detection and Analysis of Proteins Expressed from Cloned Genes. In Sambrook J, Fritsch EF, and Maniatis, T (eds), Molecular Cloning: A Laboratory Manual, Cold Spring Harbor Laboratory Press, Cold Spring Harbor, New York (1989)). Once the separation is complete, a test population of nematodes can be introduced onto the separation matrix. Behavioral preference for, or avoidance of, different regions of the matrix would indicate the presence of a substance at that location that modifies the activity of mammalian GPCR(s) expressed in the chemosensory neurons. The measurement of chemotactic behavior involves determining the spatial distribution of a test population of transgenic nematodes of the present invention at an initial time point and at a later time point when the test population is exposed to at least two spatially distinct concentrations of a substance. Methods for assaying the distribution of nematodes, e.g. *C. elegans,* have already been described (See Bargmann, supra; Troemel, *et al*., Cell 91:161-169 (1997)). An alternative to direct observation is mechanical detection of the animals. For instance, such detection could involve the determination of optical density across the test surface by a machine. The animals would be detected by changes in density at the location where an animal was located. Alternatively, if the animals are expressing a reporter gene that can be detected in living animals (ie., GFP), a machine could monitor the position of the animals using a suitable reporter gene detection protocol.

In another embodiment of the invention, determining spatial distribution of the transgenic nematodes involves assaying the feeding behavior of the animals. Soil nematodes, such as *C. elegans,* typically feed on bacteria, and it is possible to grow *C. elegans* on a variety of bacterial species (Venette, et *al.* (1998), *Soil Biology and Biochemistry* **30:** 949-960). In a preferred embodiment of the invention, such bacteria are selected from the group consisting of: *Acinefobacter calcoaceticus*, *Bacillus cereus, Bacillus sp., Enterobacter amnigenus, Enterobacter cloacae, Escherichia coli, Flavobacterium sp., Proteus vulgaris, Pseudomonas aeruginosa, Pseudomonas cepacia, Pseudomonas maltophilia, Pseudomonas putida, Pseudomonas sp., Serratia liquefaciens, Serratia marcesens, Zymobacillus macerans.* In the laboratory, strains of nematodes, including *C. elegans,* are customarily grown on uniform "lawns" of *E. coli* bacteria placed on an agarsurface (Epstein, *et al., Caenorhabditis Elegans:* Modern Biological Analysis of An Organism, Academic Press, San Diego, CA. (1995)). As the *C. elegans* grow and reproduce, the *E. coli* lawn is diminished until no bacteria remain. Therefore, the reduction of the bacterial lawn is a measurement of an actively feeding population of *C. elegans.* The rate of decrease of the lawn is proportional to the number of feeding animals present. The consumption of bacteria can be used as an indirect indicator of the distribution of animals. Substances can be spotted onto a substrate in an array, and a uniform lawn of bacteria placed on the substrate, overlaying the array. A test population of *C. elegans* can be introduced and incubated for a suitable time period to allow consumption of the lawn. If the animals are attracted to a substance, then they will move to that region of the substrate and the bacterial lawn will be diminished at that spot in comparison to the surrounding lawn. Conversely, if a substance repels the animals, then the bacterial lawn at that spot will not be consumed. Thus, bacterial consumption can be used to assay the growth and location of a population of nematodes. If a GFP-expressing bacterial strain is used, the variations in bacterial density can be easily detected, for example, by photographing fluorescence intensity.

In yet another particularly preferred embodiment of the invention, microorganismal colonies, such as bacteria, viruses or yeast, are used as the point sources of test substances. A large number of techniques exist to create "libraries" of substances expressed by microorganisms (Cesareni, et *al.*, *Comb Chem High Through Put Screen* **2(1)**: 1-17(1999); Georgiou, et al., *Nat Biotechnol.* **15(1)**: 29-34 (1997); Sambrook, *et al.,* 12. Screening Expression Libraries with Antibodies and Oligonucleotides. In Sambrook J, Fritsch EF, and Maniatis, T (eds), Molecular Cloning: A Laboratory Manual, Cold Spring Harbor Laboratory Press, Cold Spring Harbor, New York. (1989)). These libraries can be used in the inventive methods to identify specific test substances that modify activity of the mammalian GPCRs expressed in the test population of nematodes. For example, microorganismal colonies, each of which secrete a different substance, can be placed on the substrate medium. A test population of nematodes can then be introduced onto the substrate. The behavioral preference of the animals to these colonies would indicate potential activity of the substance secreted by the colony on the mammalian GPCR(s) expressed in the test population.

In another particularly preferred embodiment of the invention, chemotactic behavior of the transgenic nematodes is assayed by combining the ability of nematodes to consume bacteria with the ability to generate recombinant libraries of substances in bacteria, as discussed above. For instance, a library of secreted substances can be engineered through recombinant DNA methods in bacteria. The library can be plated out as individual colonies on an agar substrate, with each colony secreting a different substance. A test population of nematodes could then be introduced onto the substrate. With no chemotactic preference shown by the animals, the colonies can be consumed at the same rate. However, if one or more of the colonies expressed a substance that was chemorepulsive to the animals, then that colony or colonies can be consumed at a slower rate. These "resistant" colonies could then be recovered and the substance identified through analysis of the bacterial clone.

Thus, in a particularly preferred embodiment, the present invention provides a method for identifying at least one ligand of a mammalian GPCR said method comprising the steps of (a) providing at least one nematode that expresses a mammalian GPCR in the sensory neurons that are linked to behavior; (b) placing at least two different concentrations of at least one test substance onto a substrate surface that contains growth medium; (c) placing a uniform lawn of bacteria onto the surface of said growth medium; (d) contacting said at least one nematode with the said uniform lawn of bacteria; and (e) detecting, after a suitable time period, a decrease in the density of said uniform lawn of bacteria. The dauer pathway is an alternative life cycle stage common to many nematode species. The dauer pathway is normally triggered by environmental stresses such as starvation, temperature extremes, or overcrowding.

Genetically, the dauer pathway has been most intensively studied in *C. elegans.* The response to overcrowding in *C. elegans* is mediated by a substance known as dauer pheromone, which is secreted by the animals. When dauer pheromone becomes sufficiently concentrated, it triggers commitment to the dauer alternative life cycle stage. Three sensory neurons have role in the appropriate response to dauer pheromone: ADF, ASI, and ASJ neurons. In addition to pheromone, activation of a transgenically expressed mammalian GPCR in these neurons of *C. elegans* would be expected to lead to commitment to the dauer pathway. Unlike chemotaxis assays, measuring dauer formation does not require presentation of substances on a substrate that allows movement of *C. elegans,* nor does it require that the substance be present at two spatially distinct concentrations. Preferably, the test population of *C. elegans* are exposed to the substance during the portion of the life cycle at which commitment to the dauer pathway is made. Measurement of dauer formation has been previously described. Thermotaxis chemosensory behavior can be used to assay the function of mammalian GPCRs that might be affected by temperature. A test population of *C. elegans* can be generated as described above that express a mammalian GPCR in the AFD neuron. The AFD neuron mediates thermotaxis, and expression of a thermoreactive mammalian GPCR in this neuron can modify the thermotactic behavior of the test population of *C. elegans.* The test population of animals can then be placed on a solid substrate that is maintained in athermal gradient. The preference of the test population for certain thermal isoclines on the substrate can be used as a measure of mammalian GPCR activation. In a particularly preferred embodiment of the invention, the chemosensory behavior of nematodes can be used to identify substances that activate mammalian GPCRs. As outlined above, mammalian GPCRs can be expressed in nematodes, and the nematodes can then be exposed to an array of substances. By comparing the chemosensory behavior seen in test populations of wildtype animals (non-transgenic) and transgenic animals expressing a particular mammalian GPCR, substances can be identified that alter the behavior of only the transgenic nematodes. These substances would then be candidates for interacting with and/or modifying the activity of the transgenically expressed mammalian GPCR.

Thus, a particularly preferred embodiment of the invention encompasses screening for substances that can activate mammalian GPCRs that have no known ligand, which are referred to in the art as "orphan receptors". Most preferred in the methods of the present invention is the screening for substances activating mammalian olfactory, taste and vomeronasal receptors. Orphan GPCRs can be expressed in nematodes and, as outlined above, an array of test substances could be exposed to the animals. The behavior of the animals will then indicate the presence of an activating substance or ligand. In yet another preferred embodiment of the invention, the test substances are present in beads, and the medium is solid or semi-solid.

Thus, a particularly preferred embodiment of the present invention provides a method for identifying at least one ligand, agonist or modulator of at least one mammalian GPCR, said method comprising the steps of: (a) providing at least one nematode that expresses a mammalian GPCR in the sensory neurons that are linked to behavior; (b) placing a medium in a container; (c) placing at least two different concentrations of at least one test substance in said container; (d) adding said at least one nematode to said container; and (e) detecting, after a suitable time period, the behavioral response of said at least one nematode over the surface of said medium. Using this approach, the present invention would allow the inexpensive testing of a large number of substances. For example, once a transgenic strain of nematode is generated, large populations of transgenic nematode can be grown for chemotactic assays. Many substances can be tested by spotting them in a large array on the substrate medium, as discussed above. In addition, a large variety of substances created by recombinant DNA techniques in microorganisms can be assayed at once. Thus, the invention can be used to identify rare or novel substances that act on certain mammalian GPCRs. This ability will have special utility when analyzing the mammalian GPCRs identified only through analysis of DNA sequence.

In a particularly preferred embodiment of the invention, screening methods with the transgenic nematodes can be used to evaluate the potency and specificity of substances on mammalian GPCR activity. A strain of nematodes can be generated that expresses a particular mammalian GPCR upon which a substance is known to act. Structurally related substances could then be tested and compared in a chemotactic assay for their efficacy in eliciting a chemotactic response from the mammalian GPCR-expressing strain. Because chemotactic response is a result of receptor activation, the response can be used as a gauge for how well substances can activate the mammalian GPCR. For instance, structurally related compounds can be tested to determine which chemical differences are important in activating the mammalian GPCR and eliciting a chemosensory response. Such information is important in determining the structure/activity relationship of different substances, and can lead to an understanding of what part of the chemical structure is important for receptor activity.

In a particularly preferred embodiment, the present invention provides a method for evaluating the potency of mammalian GPCR activation by a known ligand, said method comprising the steps of: (a) providing at least one nematode that expresses at least mammalian GPCR, in sensory neurons that are linked to behavior, wherein said mammalian GPCR has a known ligand; (b) contacting said at least one nematode with said ligand and at least one structurally related substance; and (c) detecting the behavioral response of said at least one nematode to said at least one structurally related substance; and (d) comparing the behavioral response of said at least one nematode to said ligand to the behavioral response of said at least one nematode to said at least one structurally related substance.

The term, "structurally related substances," as it is used herein, refers to substances which share a common or core structure of the substance that has biological activity, such as a common pharmacophore or olfactophore. Such "structurally related substances" can differ from each other, however, in their substituent groups. As used in the screening assays herein, "structurally related substances" share a common structure such as a pharmacophore or olfactophore with a known or newly discovered ligand of a non-nematode cell surface receptor that is expressed in the transgenic nematodes of the present invention.

In a particularly preferred embodiment of the methods of the invention, the mammalian GPCR is a mammalian taste, olfactory or vomeronasal receptor. In another particularly preferred embodiment of the present invention, the nematode is *C. elegans.*

The present invention also provides for the use of any one of the methods described above for the identification of an agonist, an antagonist or a modulator of a mammalian taste, olfactory or vomeronasal receptor.

The substances identified by the methods of the present invention as ligands, antagonists or modulators of olfactory, taste, and vomeronasal receptors and particularly orphan olfactory, taste, and vomeronasal receptors may be used in a number of different compositions and products as "improving mood" molecules, anti-stress, ansiolytics, fragrance and perfume in fragrant products including consumer care products, taste in food or beverage products, antagonists or masking of "bad odor", antagonists or masking of off-tastes, taste or fragrance modulators etc. For example ligands identified for the family of T1R taste receptors (mediating sweet taste) could serve as better sweeteners that the ones currently available. In the same way compounds antagonizing those ligands can be used to block or modify the sweet taste if necessary. Antagonists of the T2R family of taste receptors (detecting bitter taste) blocking or modifying bitter taste would be useful in many pharmacological and nutritional preparations, in medicaments, health products, food products and beverage products. Substances identified as agonists, antagonists, or modifiers of sweet, bitter and umami receptors may be used in the above-mentioned preparations and products to achieve an adequately balanced taste perception.

Monosodium glutamate produces umami sensation but has some inconveniences, therefore substances identified producing umami sensation are required. It is believed that the umami taste is mediated by a truncated form of mGluR4, a metabotropic GPCR (for review see Lindemann (2001), *Nature* **413**: 219-225). Umami taste is also thought to be mediated by the human T1R1/T1R3 heterodimer (T1R is the family of taste receptors that detect sweet sensation) (see Li et al. (2002), *Proc. Natl. Acad. Sci. USA* **99**: 4692-4696).

Therefore, in a further embodiment of the present invention, a method of producing a composition comprising the steps of the methods described above, modifying the identified substance and formulating the substance obtained with an acceptable carrier or diluent, is provided.

Depending on the intended use of the composition, adequate carriers or diluents have to be added. Examples of such carriers and methods of formulation for pharmaceutical compositions may be found in Remington's Pharmaceutical Sciences. To form a pharmaceutically acceptable composition suitable for effective administration, such compositions will contain an effective amount of the identified substance. Carriers may serve as solvents for the identified water-soluble or volatile substances. Moreover, when desired or necessary, suitable binders, lubricants, disintegrating agents and coloring agents can also be incorporated into the composition. Examples of such carriers or diluents for flavor or fragrance compounds may be found e.g. in "Perfume and Flavor Materials of Natural Origin", S. Arctander, Ed., Elizabeth, N.J., 1960, in "Perfume and Flavor Chemicals", S. Arctander, Ed., Vol. I & II, Allured Publishing Corporation, Carol Stream, USA, 1994, in "Flavourings", E. Ziegler and H. Ziegler (ed.), Wiley-VCH Weinheim, 1998, and "CTFA Cosmetic Ingredient Handbook", J.M. Nikitakis (ed.), 1$^{st}$ ed., The Cosmetic, Toiletry and Fragrance Association, Inc., Washington, 1988. Carriers include encapsulating agents and gums such as maltodextrin, gum arabic, alginates, gelatin, modified starch, and polysaccharides. The compounds may be formulated in form of a solution. Solvents which maybe used are known to those skilled in the art and include e.g. ethanol, ethylene glycol, propylene glycol, glycerin, triacetin, diethyl phthalate and dimethyl phthalate. Fragrance or flavor compositions may comprise other ingredients normally used in the formulation of said compositions. Such ingredients are known to those skilled in the art and include e.g. antifoaming agents, antimicrobial agents, antioxidants, antiredeposition agents, bleaches, colorants, emulsifiers, enzymes, fats, fluorescent materials, fungicides, hydrotropes, moisturizers, optical brighteners, perfume carriers, perfume, preservatives, proteins, silicones, soil release agents, solubilizers, sugar derivatives, sun screens, surfactants, vitamins, waxes, etc. To form a acceptable composition suitable for use as flavor or fragrance, respectively, such compositions will contain an effective amount of the identified substance.

The substances identified by the method of the invention may be modified to achieve (i) modified site of action, spectrum of activity, and/or (ii) improved potency, and/or (iii) decreased toxicity (improved therapeutic index), and/or (iv) decreased side effects, and/or (v) modified onset of action, duration of effect, and/or (vi) modified kinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carbon acids, or (iii) esterification of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiozolidines or combinations thereof; and (b) formulating the product of said modification with a pharmaceutically acceptable carrier or a carrier/diluent acceptable for fragrance or flavor compositions or products.

In summary, the invention proposes that expression of mammalian GPCRs within the neurons of nematodes can create behavioral phenotypes. These behavioral phenotypes are useful for the identification of agonists, antagonists and modulators that act on the mammalian GPCRs, especially on olfactory, taste or vomeronasal receptors. In addition, the behavioral phenotypes are useful for the evaluation of specificity and efficacy of substances that interact with mammalian GPCRs.

Having now generally described this invention, the same will become better understood by reference to the specific examples, which are included herein for purpose of illustration only and are not intended to be limiting unless otherwise specified, in connection with the following figures.

## Figures

**Figure 1**: Schematical drawing of gene expression construct pPA1.

**Figure 2**: Schematical drawing of gene expression construct pPA2.

**Figure 3**: A, Chemotaxis assays on adult *C. elegans* to volatile compounds. Population chemotaxis assays were conducted on round plates divided in 6 zones labeled 1-6. One microliter of odorant and one microliter 1 M sodium azide were added at zone 1, and one microliter of control solvent and one microliter 1 M sodium azide were added at zone 6. Fifty young adult *C. elegans* were placed in the centre of the plate and counted after 1 hour to determine the chemotaxis index (see Experimental Procedures). B, Chemotaxis assays on adult *C. elegans* to soluble compounds. Population chemotaxis assays were conducted on round plates divided in 2 zones labeled 1 and 2. Different concentrations of the soluble compounds were added in the buffered CTX agar of zone 1 and control solvent was added at zone 2. Fifty young adult *C. elegans* were placed in the centre of the plate and counted after 1 hour to determine the chemotaxis index (see Experimental Procedures).

**Figure 4**: Wild-type *C. elegans* are responsive to the odorant octanal. A, histogram showing the analysis of the chemotaxis response of wild-type worms to octanal. Worms were exposed to one microliter of undiluted octanal or to one microliter from a series of octanal dilutions as indicated below each column and in Experimental Procedures. Volatile attraction or avoidance was observed depending on octanal concentration. Each data point represents the mean ± SEM of eight independent experiments. B, chemical structure of octanal (octyl aldeyde).

**Figure 5**: Expression of rat I7 in AWB neurons decrease the volatile attraction response of *C. elegans* to octanal 1:10. A, histogram shows changes in the chemotaxis responses of five different Str-1::I7 transgenic worm strains to octanal 1:10 relative to wild-type worms. Chemotaxis essays were performed as indicated in Experimental Procedures. Each data point represents the mean ± SEM of eight independent experiments. B, histogram showing the distribution per zones of wild-type and Str-1::I7-1 transgenic worms. Each data point represent the mean ± SEM of eight independent experiments. *White bars,* wild-type worms. *Black bars,* transgenic worms. *Asterisks* denote values that differ significantly in transgenic worms relative to wild-type worms ($^{***}$ $p<0.001$, ** $p<0.01$, *<0.02). C, rat 17 is expressed in Str-1::I7 transgenic worms. 17 transcript was amplified by PCR from total RNA after (+) and before (-) reverse transcription, and from plasmid containing I7 as a positive control. *Bottom panel,* Ama-1 transcript was amplified in parallel from total RNA after (+) and before (-) reverse transcription, and from *C. elegans* genomic DNA as a positive control. M, molecular size markers; WT, wild-type worms; C, positive control; $H_2O$, negative control.

**Figure 6:** Expression of rat I7 in AWA neurons decreases the volatile avoidance response of *C. elegans* to undiluted octanal. A, histogram shows changes in the chemotaxis responses of four different Odr-10::I7 transgenic worm strains to undiluted octanal relative to wild-type worms. Each data point represents the mean ± SEM of eight independent experiments. B, histogram showing the distribution per zones of wild-type and Odr-10::I7-1 transgenic worms. Each data point represents the mean ± SEM of eight independent experiments. *White bars,* wild-type worms. *Black bars,* transgenic worms. *Asterisks* denote values that differ significantly in transgenic worms relative to wild-type worms ($^{***}$ $p<0.001$, $^{**}$ $p<0.01$, *<0.02). C, rat I7 is expressed in Odr-10:: I7 transgenic worms. I7 transcript was amplified by PCR from total RNA after (+) and before (-) reverse transcription, and from a plasmid containing I7 cDNA as a positive control. *Bottom panel,* Ama-1 transcript was amplified in parallel from total RNA after (+) and before (-) reverse transcription, and from *C. elegans* genomic DNA as a positive control. M, molecular size markers; WT, wild-type worms; C, positive control; $H_2O$, negative control.

**Figure 7**: Gene expression construct pPA3 for the human T2R4 bitter taste receptor (Adler, E, Hoon, MA, Mueller, KL, Chandrashekar, J, Ryba, NJ and Zuker, CS (2000) A novel family of mammalian taste receptors. Cell 100, 693-702; GenBank accession number: AF227131) in the ASI neurons of C. elegans.

**Figure 8:** Wild-type C. elegans are responsive to the bitter tastant 6-n-propyl-2-thiouracil (PROP). Histogram showing the analysis of the chemotaxis response of wild-type worms to PROP. Worms were exposed to 1mM, 2.5mM, and 5mM concentrations of PROP. Avoidance was observed at PROP concentrations of 2.5mM and 5mM. Each data point represents the mean +/- SD of eight independent experiments.

**Figure 9:** Expression of human T2R4 bitter taste receptor (Chandrashekar, J, Mueller, KL, Hoon, MA, Adler, E, Feng, L, Guo, W, Zuker, CS and Ryba, NJ (2000) T2Rs function as bitter taste receptors. Cell 100, 703-711) in ASI neurons decreases the volatile avoidance response of C. elegans to 5mM PROP. Histogram shows changes in the chemotaxis responses of three different gpa-4::T2R4 transgenic worm strains to 5mM PROP relative to wild-type worms. Each data point represents the mean +/- SD of eight independent experiments.

## Examples

Commercially available reagents referred to in the Examples were used according to manufacturer's instructionsand/or general methods according to Sambrook, *et al.*, 1. Plasmid Vectors. In Sambrook J, Fritsch EF, and Maniatis, T (eds), Molecular Cloning: A Laboratory Manual, Cold Spring Harbor Laboratory Press, Cold Spring Harbor, New York. (1989) and F.M. Ausubel, R. Brent, R.E. Kingston, D.D. Moore, J.G. Seidman, J.A. Smith, K. Struhl (eds). Current Protocols in Molecular Biology. Current Protocols 1987-1994; John Wiley & Sons, Inc. 1994-1998.

**Example 1** Generation of transgenic worms

Wild-type nematodes were *C. elegans* variety Bristol, strain N2 (obtained from the Caenorhabditis Genetics Center). Worms were maintained at 20°C using standard methods (Brenner, S. (1974) Genetics 77, 71-94). Transgenic worms were generated by injecting the DNA solution into the central core of cytoplasm in each arm of the hermaphrodite gonad. (Mello, C.C., Kramer, J.M., Stinchcomb, D., and Ambros, V. (1991) EMBO J. 10, 3959-3970). 0.5 pg of the GFP gut marker pcl2 DNA and 0.5-1 pg of pPA1 (Fig. 1) or pPA2 (Fig. 2) or pPA3 (Fig. 7) DNA were coinjected into gonads of hermaphrodite worms. Transformed animals were allowed to self-fertilize and first-generation of individuals showing heritably extrachromosomal transformation were selected. Transgenic animals were identified by expression of GFP in the gut.

Deposition Data: The rat 17 receptor (SEQ ID NO: 1) clones pPA1 and pPA2 were deposited under the Budapest Treaty at the Deutsche Sammlung von Microorganismen und Zellkulturen GmbH (DSMZ), Mascheroder Weg 1 b, D-38124 Braunschweig, Germany, with an effective deposition date of 19th July 2002 under the accession numbers DSM 15106 (pPA1) and DSM 15107 (pPA2), respectively. The human T2R4 receptor (SEQ ID NO: 6) clone pPA3 was deposited under the Budapest Treaty at the Deutsche Sammlung von Microorganismen und Zellkulturen GmbH (DSMZ), Mascheroder Weg 1 b, D-38124 Braunschweig, Germany, with an effective deposition date of 13th June 2003 under the accession number DSM 15674 (pPA3).

**Example 2** Reverse transcription-polymerase chain reaction (RT-PCR) analysis of 17 gene expression

Total RNA was prepared from mixed stage worm samples purified by sucrose flotation. The worms were mechanically disrupted in RNAzol B (Tel-Test, Inc, Friendswood, TX) using FastRNA beads and a FastPrep Machine (Qbiogene, Carlsbad, CA). Total RNA was extracted using the MagNA Pure LC RNA isolation Kit I and a MagNA Pure LC Instrument (Roche Molecular Biochemicals). First-strand cDNA was prepared from 50 ng of total RNA using 1 mM p(DT)15 primers (Roche Molecular Biochemicals) and the Sensiscript Reverse Transcriptase kit (QIAGEN, Hilden, Germany). This cDNA was used as template for PCR amplifications that were performed with the following primer pairs: I7-24 (SEQ ID NO: 2) and I7-649 (SEQ ID NO: 3) for I7 cDNA amplification (product size: 626 bp) and ama-1 (SEQ ID NO: 4) and ama-1 (SEQ ID NO: 5) for ama-1 cDNA amplification (product size: 522 bp or 426 bp, using genomic DNA or cDNA as template respectively). PCR reactions were performed in a total volume of 100µl containing 50 ng of plasmid DNA, 200nM of each primer in 50 mM KCl, 10mM Tris (pH 8.0), 1.5 mM $MgCl_2$, 50 mM of each dNTP, and 2.5U of Taq DNA polymerase primers (Roche Molecular Biochemicals, Mannheim, Germany). An initial denaturation step at 94°C for 2 min was followed by 35 cycles of denaturation at 94°C for 30 s, annealing at 55° C for 30 s, and extension at 72°C for 1 min on a Mastercycler gradient PCR machine (Eppendorf-Netheler-Hinz, Hamburg, Germany). The last extension step was at 72°C for 4 min. When no band was detected following 35 cycles, the PCR reaction was subjected to 15 additional cycles. As a negative control, total RNA extracted from worms was treated in the same way, except that no reverse transcriptase was added. The PCR products were analysed on agarose gels stained with ethidium bromide.

**Example 3** Behavioural Assays for volatile substances

Population chemotaxis assays, in which attraction or avoidance to an odorant was measured by establishing a gradient of the volatile chemical from a point source (Fig. 3A), were performed on round 9 cm agar plates divided in 6 zones labelled 1-6. (Bargmann, C.I., Hartwieg, E., and Horvitz, H.R. (1993) *Cell* **74**, 515-527). One microliter of odorant and one microliter 1 M sodium azide were added at one end of the dish (zone 1), and one microliter of solvent (ethanol) and one microliter 1 M sodium azide were added at the opposite end (zone 6) as a control. Sodium azide was included to anaesthetize animals entering zones 1 and 6. Fifty young adult trangenic *C. elegans,* or fifty young adult wild-type *C. elegans* (as a control), were deposited in the centre of the plate and scored based on their final position on the plate after 1 hour. The Chemotaxis Index (C. I.) was calculated using the formula:

$$C.I.= ((1+2) - (5+6)) / N$$

where 1, 2, 5, and 6 are the number of worms in the corresponding zones of the plate, and N is the total number of worms in all six zones of the dish. A chemotaxis index of+1.0 indicates total attraction, an index of-1.0 indicates total repulsion. Where reported, worms were exposed to a 1:100 dilution of diacetyl, undiluted benzaldehyde, undiluted octanal and 1:5, 1:10, 1:15, 1:25, 1:50, 1:100, and 1:1000 dilutions of octanal (Sigma Chemical Co., St. Louis, MO). Population assay data for N2 and transgenic worm lines were compared using the two-tailed t test.

To investigate the possibility to functionally express mammalian ORs in *C. elegans,* wild-type nematodes were first characterized for their ability to detect and respond to increasing dilutions of volatile chemicals and particularly to octanal, an agonist for the rat I7 OR (Fig. 4B; Zhao, H., Ivic, L., Otaki J.M., Hashimoto, M., Mikoshiba, K., and Firestein, S. (1998) *Science* **279**, 327-242; Araneda, R.C., Kini, A. D., and Firestein, S. (2000) *Nature Neurosci.* **12**, 1248-1255). As shown in Fig. 4A, undiluted octanal was repellent for wild-type worms (chemotaxis index -0.6) whereas a 1:10 dilution was attractant (chemotaxis index +0.5). Higher dilutions of octanal gave chemotaxis indexes close to 0 indicating a neutral response of the wild-type worms to lower concentrations of the odorant. These results clearly showed that wild-type worms are responsive to octanal and identified the optimal dilutions of octanal to be used in the next set of experiments with rat 17-transgenic *C. elegans.* As AWB neurons mediate volatile avoidance in *C. elegans,* it was exprected that the expression of the rat 17 OR in AWB neurons and its activation with a 1:10 dilution of octanal would counteract the olfactory attraction to 1:10 octanal observed in wild-type worms. 17 transgenic worms were generated, in which 17 expression was driven by the *str-1* promoter, a controlling element exclusively functional in the two AWB neurons (Troemel, E.R., Kimmel, B.E., and Bargmann, C.I. (1997) *Cell* **91**, 161-169). Five independently derived *C. elegans* transgenic strains expressing 17 were tested (Fig. 5). As shown in Fig. 5A, the chemotaxis index for 1:10 octanal decreased dramatically in the five Str-1::I7 transgenic worm strains in relation to control wild-type worms. The expected shift from attraction to neutrality in the behavioral response of the worms to 1:10 octanal was statistically significant in our five *C. elegans* transgenic strains (Fig. 5A), all of which expressed I7 (Fig. 5C). While most of the wild-type *C. elegans* were attracted to zone 1, where the odorant source was located, a significant number of str-1 :: I7 transgenic worms was found in distant zones to the odorant source (Figs. 5B and 3A). To investigate the specificity of the shift from attraction to neutrality in the behavioral response of Str-1::I7 transgenic worms upon octanal exposure, behavioral chemotaxis assays with Str-1 ::I7 transgenic worms were performed in which we used a 1:100 dilution of diacetyl, an odorant known to induce attraction in wild-type *C. elegans* (Troemel, E.R., Kimmel, B.E., and Bargmann, C.I. (1997) *Cell* **91**, 161-169). It was found that the chemotaxis index for diacetyl did not change in the Str-1 ::I7 transgenic worms in relation to control wild-type worms. To further confirm that the decrease of the chemotaxis index for octanal in Str-1 ::I7 transgenic worms was due to the interaction of octanal with I7, Str-1::I7 worms were expossed to pure benzaldehyde, an aromatic aldehyde known to do not activate the I7 receptor (Araneda, R.C:, Kini, A.D. and Firestein, S. (2000) *Nature Neurosci.* **12**, 1248-55) and that, at this concentration, induce aversion in wild-type *C. elegans* (Bargmann C.I., Hartwieg, E., and Horvitz, H.R. (1993) *Cell* **74**, 515-27). Again, the chemotaxis index for benzaldehyde did not change in the Str-1::I7 transgenic worms in relation to control wild-type worms.

AWA neurons mediate chemotaxis to volatile attractants in C. *elegans.* To determine whether the 17 receptor would also function in AWA neurons and induce attraction, 17 transgenic *C. elegans* expressing I7 under the control of the Odr-10 promoter, a controlling element exclusively functional in the two AWA neurons, were generated (Sengupta, P., Chou, J.H., Bargmann, C.I. (1996) *Cell* **84**, 875-887). It was expected that the activation of I7 with undiluted octanal in these transgenic worms would counteract the volatile avoidance to undiluted octanal observed in wild-type worms. Chemotaxis assays were performed with four independently derived *C. elegans* transgenic strains expressing I7 (Fig. 6). As shown in Fig. 6A, the chemotaxis index for undiluted octanal increased dramatically in the four Odr-10::I7 transgenic worm strains analyzed in relation to control wild-type worms. As expected, Odr-10::I7 transgenic *C. elegans* expressed 17 and showed a statistically significant decrease in their aversive response to undiluted octanal (Fig. 6A and C). While most of the wild-type *C. elegans* were repelled toward zone 6, a significant number of Odr-10::I7 transgenic worms was found in the proximity to the odorant source (Figs. 6B and 3A). In order to investigate the specificity of the decrease in the aversive response of Odr-10::I7 transgenic worms to undiluted octanal and to confirm that this decrease was due to the interaction of octanal with I7, chemotaxis assays with Odr-10::I7 transgenic worms were performed in which diacetyl and benzaldehyde were used. The results showed that the chemotaxis indexes for diacetyl and benzaldehyde did not change in the Odr-10::I7 transgenic worms in relation to control wild-type worms.

**Example 4:** Behavioral Assays for soluble substances

Nematode population chemotaxis assays for soluble compounds were assessed on round 9 cm agar plates divided in 2 zones which are separated by a plastic spacer (Fig. 3B). Each zone is filled with 10ml of buffered CTX agar (2%agar, 1mM $CaCl2$, 1mM $MgSO4$ and 5mM $KPO4$ pH 6), containing either a dissolved compound (zone 1) or no compound (zone 2). The spacer is located into the agar to separate the agar containing the chemical compound and the agar without chemical compound but the spacer is not in contact with the worms, which are on the surface of the agar. A population of animals was washed two times in a CTX buffer (1mM $CaCl2$, 1mM $MgSO4$ and 5mM $KPO4$ pH 6). Fifty young adult *C. elegans* were placed on the surface of the agar in the centre of the plate and scored based on their final position on the plate after 1 hour. The Chemotaxis Index (C. I.) was calculated using the formula:

$$C.I. = (1-2) / N$$

where 1, 2 are the number of worms in the corresponding zones of the plate, and N is the total number of worms in both zones of the plate. A chemotaxis index of +1.0 indicates total attraction, an index of-1.0 indicates total repulsion.

In order to establish a fast and reliable system to functionally express mammalian taste receptors the possibility to functionally express mammalian taste receptors in the nematode was investigated. Wild-type *C. elegans* were characterized for their ability to detect and respond to different concentrations of water-soluble chemicals including PROP (Figure 8). Population chemotaxis assays were performed on round 9 cm agar plates divided in 2 zones which are separated by a plastic spacer (Figure 3B). Each zone is filled with 10ml of buffered CTX agar (2% agar, 1mM $CaCl2$, 1mM $MgSO4$ and 5mM $KPO4$ pH 6), containing either a dissolved compound (zone 1) or no compound (zone 2). The spacer is located into the agar to separate the agar containing the chemical compound and the agar without chemical compound but the spacer is not in contact with the worms, which are on the surface of the agar. A population of animals was washed two times in a CTX buffer (1mM $CaCl2$, 1mM $MgSO4$ and 5mM $KPO4$ pH 6). Fifty young adult *C. elegans* were placed on the surface of the agar in the centre of the plate and scored based on their final position on the plate after 30-60 minutes. The Chemotaxis Index (C. I.) was calculated using the formula:

$$C.I. = (1-2) / N$$

where 1, 2 are the number of worms in the corresponding zones of the plate, and N is the total number of worms in both zones of the plate. A chemotaxis index of +1.0 indicates total attraction, an index of-1.0 indicates total repulsion.

As shown in Figure 8, distinct concentrations of the bitter tastant PROP, were repellent for wild-type worms. The results clearly showed that wild-type worms are responsive to bitter compounds and identified the optimal dilutions of PROP to be used in experiments with transgenic worms. To target the expression of taste receptors to ASI *C. elegans* amphid sensory neurons mediating water-soluble chemotaxis, the gpa-4 promoter was cloned and a vector prepared containing this promoter (Figure 7). As ASI neurons mediate water-soluble attraction in *C. elegans,* it was assumed that the expression of the human T2R4 taste receptor (SEQ ID NO: 6) in ASI neurons and its activation with PROP would counteract the repulsion to PROP observed in wild-type worms. T2R4 transgenic worms were generated in which T2R4 expression was driven by the gpa-4 promoter, a controlling element exclusively functional in the two ASI neurons. *C. elegans* transgenic strains expressing human T2R4 were tested. As shown in Figure 9, the chemotaxis index for PROP decreased in the gpa-4::T2R4 transgenic worm strains in relation to control wild-type worms. The differences in the behavioral response of wild-type and transgenic worms to PROP were statistically significant. These results indicate that it is possible to functionally express mammalian taste receptors in *C. elegans.*

## Claims

1. A method for generating a transgenic nematode expressing at least one mammalian GPCR in a sensory neuron linked to behavior, wherein said method comprises the following steps:

(a) preparing at least one gene expression construct comprising a nucleotide sequence encoding a mammalian GPCR operably linked to a promotor specific for sensory neurons,
(b) generating a transgenic nematode by introducing the said at least one gene expression construct into the nematode and
(c) examining if the resulting nematode is a transgenic nematode expressing the at least one mammalian GPCR.

**2.** A method for generating a transgenic nematode expressing at least one mammalian GPCR in a sensory neuron linked to behavior, wherein said method comprises the following steps:

(a) preparing at least one gene expression construct comprising a nucleotide sequence encoding a mammalian GPCR operably linked to a promotor specific for sensory neurons and a further gene expression construct comprising a nucleotide sequence encoding an accessory protein operably linked to a promotor specific for sensory neurons,
(b) generating a transgenic nematode by introducing the at least one gene expression construct encoding a mammalian GPCR and the gene expression construct encoding an accessory protein into the nematode and
(c) examining if the resulting nematode is a transgenic nematode co-expressing the at least one mammalian GPCR and the accessory protein.

**3.** The method according to claim 2, wherein the accessory protein is a G-protein or a chaperone.

**4.** The method according to claims 1 to 3, wherein the mammalian GPCR is a mammalian taste, olfactory or vomeronasal GPCR.

**5.** The method according to claims 1 to 4, wherein the promotor specific for a sensory neuron is selected from the group comprising *daf-7,flp-6, gpa-2, gpa-4, gpa-11, gcy-5, gcy-6, gcy-7, gcy-8, nhr-38, nhr-79, odr-7, odr-10, ops-1, str-1, str-2, str-3, sra-7, sra-9, srg-2,* and *srg-8.*

**6.** The method according to claims 1 to 5, wherein the nematode belongs to the subgenus *Caenorhabditis.*

**7.** The method according to claims 1 to 6, wherein the nematode is *Caenorhabditis elegans.*

**8.** The method according to claims 1 to 7, wherein it is further determined whether the transgenic nematode shows a different behavioral phenotype selected from the group comprising chemoattraction towards or chemorepulsion from a substance as compared to wild-type nematodes.

**9.** A transgenic nematode expressing a mammalian GPCR in a sensory neuron linked to behavior.

**10.** A transgenic nematode co-expressing a mammalian GPCR and an accessory protein in a sensory neuron linked to behavior.

**11.** The transgenic nematode according to claims 9 and 10, wherein the mammalian GPCR is a mammalian taste, olfactory or vomeronasal GPCR.

**12.** A transgenic nematode generated by any of the methods according to claims 1 to 8.

**13.** A method for identifying at least one test substance that is a ligand of at least one mammalian GPCR, said method comprising the steps of

(a) providing at least one transgenic nematode that expresses at least one mammalian GPCR in sensory neurons linked to behavior;
(b) contacting said at least one nematode with at least one test substance; and
(c) detecting modulation of behavior of said at least one nematode in response to said at least one test substance.

**14.** A method for identifying at least one test substance that is an agonist of a mammalian GPCR, said method comprising the steps of

(a) providing at least one nematode that expresses at least one mammalian GPCR in sensory neurons that are linked to behavior;
(b) contacting said at least one transgenic nematode with the at least one test substance;
(c) determining whether said at least one test substance causes an activation of the said behavior in said at least one transgenic nematode; and
(d) identifying said at least one test substance that causes an activation of said behavior in said at least one transgenic nematode as an agonist of said mammalian GPCR.

**15.** A method for identifying at least one test substance that is an antagonist of a mammalian GPCR, said method comprising the steps of

(a) providing at least one nematode that expresses at least one mammalian GPCR in sensory neurons that are linked to behavior, wherein said at least one mammalian GPCR has a known agonist which activates said behavior;
(b) contacting said at least one transgenic nematode with the at least one test substance in the presence of the known agonist;
(c) determining whether said at least one test substance causes a suppression of the said behavior in said at least one transgenic nematode; and
(d) identifying said at least one test substance that causes a suppression of said behavior in said at least one transgenic nematode as an antagonist of said mammalian GPCR.

**16.** A method for identifying at least one test substance that is a modulator of a mammalian GPCR, said method comprising the steps of

(a) providing at least one nematode that expresses at least one mammalian GPCR in sensory neurons that are linked to behavior, wherein said at least one mammalian GPCR has a known agonist or antagonist which activates or inhibits said behavior;
(b) contacting said at least one transgenic nematode with the at least one test substance in the presence of the known agonist or antagonist;
(c) determining whether said at least one test substance causes a modulation of the said behavior in said at least one transgenic nematode; and
(d) identifying said at least one test substance that causes a modulation of said behavior in said at least one transgenic nematode as an modulator of said mammalian GPCR.

**17.** The method according to claims 13 to 16, wherein step (b) comprises placing a medium in a container; placing the at least one test substance on said medium; adding said at least one nematode to said container; and detecting, after a suitable time period, the behavioral response of said at least one nematode over the surface of said medium.

**18.** The method according to claim 17, wherein the test substance is a water-soluble chemotactic substance and is placed on the medium in a defined zone of the container.

**19.** The method according to claim 17, wherein the test substance is a volatile chemotactic substance and is placed on the medium at one end of the container.

**20.** The method according to claims 13 to 19, wherein the test substance is provided at at least two different concentrations.

**21.** The method according to claims 13 to 20, wherein the behavior is selected from the group consisting of water-soluble chemoattractive behavior and volatile chemoattractive behavior, and wherein the substance is a chemoattractant substance.

**22.** The method according to claims 13 to 20, wherein the behavior is selected from the group consisting of water-soluble chemorepulsive behavior and volatile chemorepulsive behavior, and wherein the substance is a chemorepulsant substance.

**23.** A method for evaluating the potency of mammalian GPCR activation by a known ligand, said method comprising the steps of

(a) providing at least one nematode that expresses at least one mammalian GPCR in sensory neurons that are linked to behavior, wherein said at least one mammalian GPCR has a known ligand;
(b) contacting said at least one nematode with said ligand and at least one structurally related substance; and
(c) detecting the behavioral response of said at least one nematode to said at least one structurally related substance; and
(d) comparing the behavioral response of said at least one nematode to said ligand to the behavioral response of said at least one nematode to said at least one structurally related substance.

**24.** The method according to claims 13 to 23, wherein the mammalian GPCR is a mammalian taste, olfactory or vomeronasal receptor.

**25.** The method according to claims 13 to 24, wherein the nematode is *C. elegans.*

**26.** Use of the method according to any one of claims 13 to 25 for the identification or evaluation of an agonist, an antagonist or a modulator for a mammalian taste, olfactory or vomeronasal receptor.

**27.** A method of producing a composition comprising the steps of any one of claims 13 to 25, modifying the identified substance and formulating the substance obtained with an acceptable carrier or diluent.

**28.** The method substantially as herein before described especially with reference to the foregoing Examples.

Figure 1

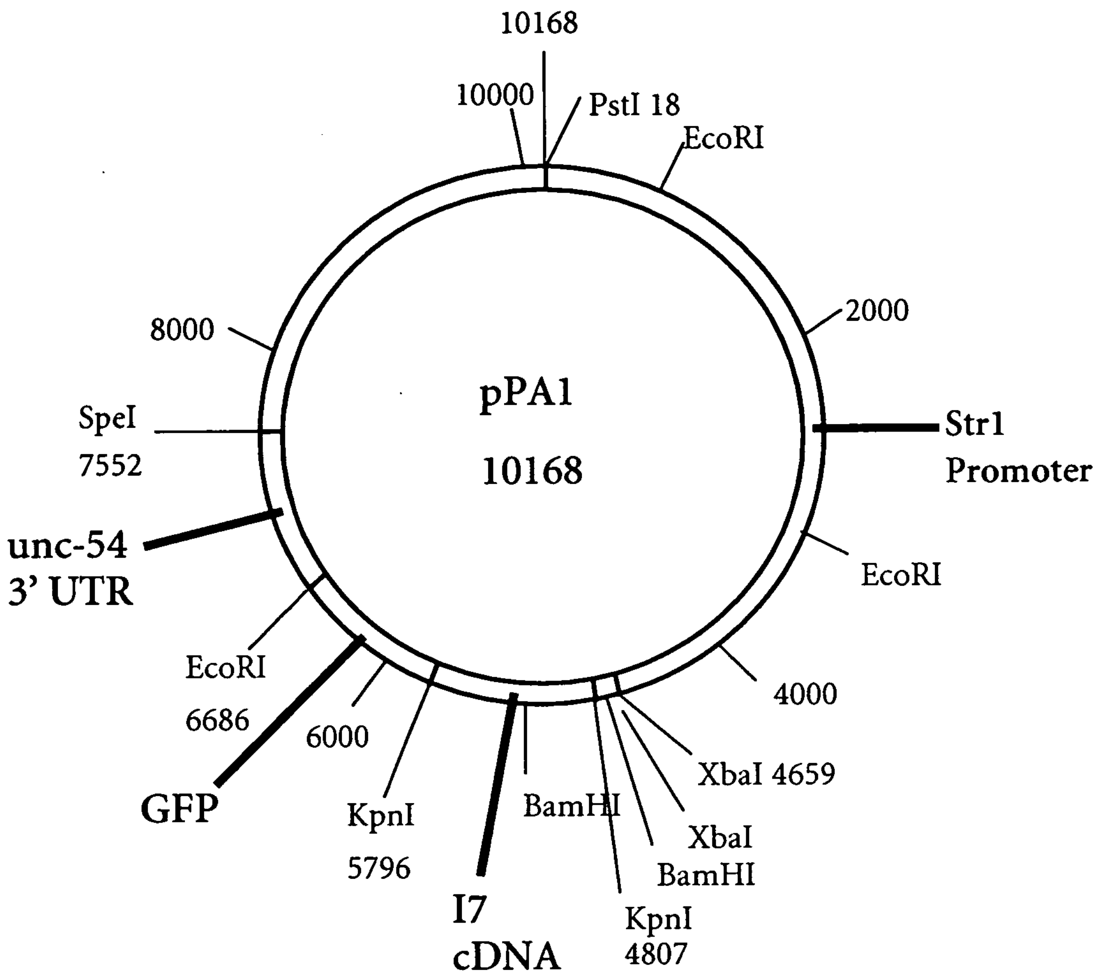
10168
10000
PstI 18
EcoRI
2000
Str1
Promoter
EcoRI
4000
XbaI 4659
XbaI
BamHI
KpnI
4807
BamHI
I7
cDNA
KpnI
5796
6000
GFP
EcoRI
6686
unc-54
3' UTR
SpeI
7552
8000
pPA1
10168

Figure 2

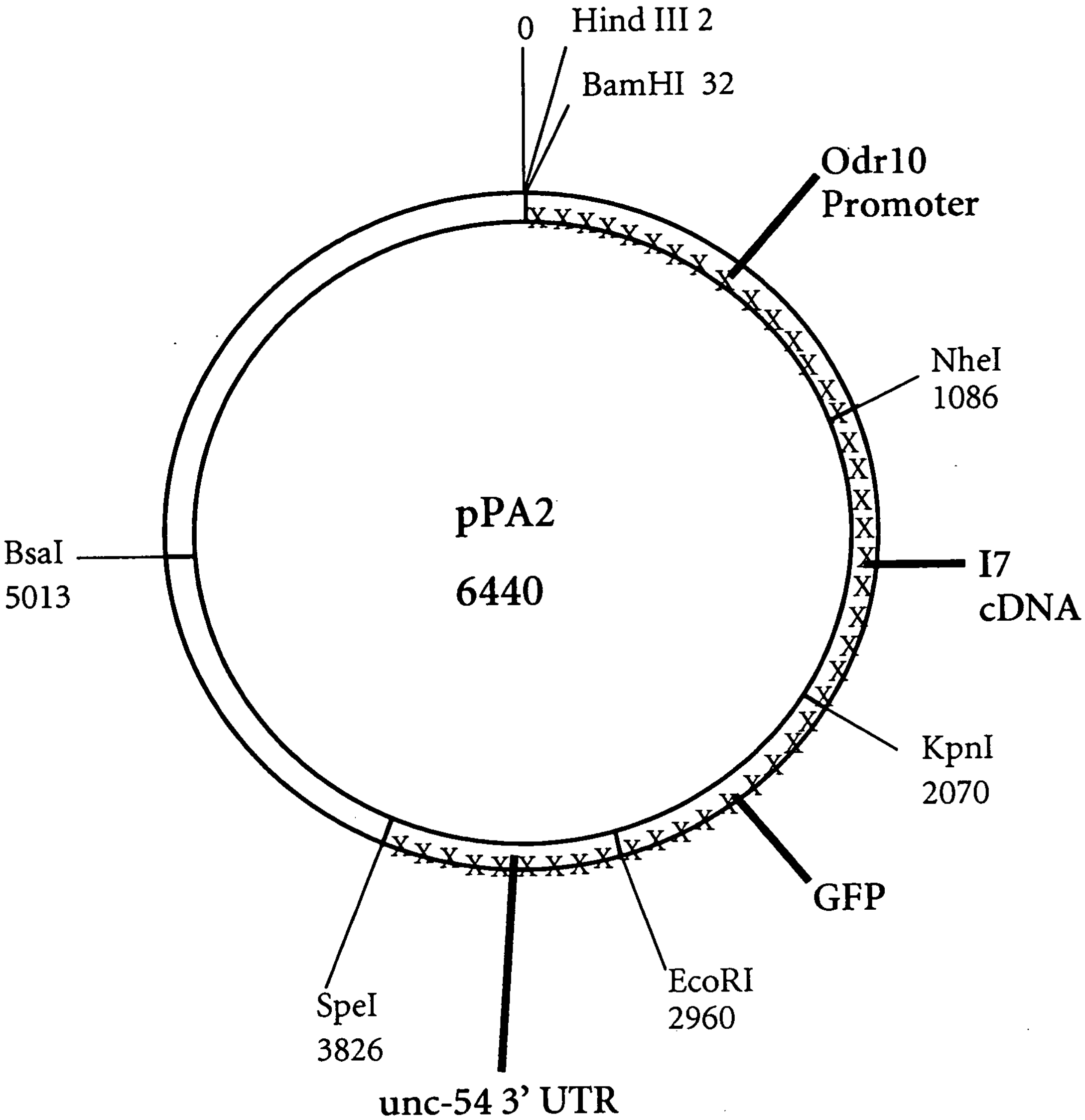
0
Hind III 2
BamHI 32
Odr10 Promoter
NheI 1086
I7 cDNA
KpnI 2070
GFP
EcoRI 2960
unc-54 3' UTR
SpeI 3826
BsaI 5013
pPA2
6440

Figure 3A

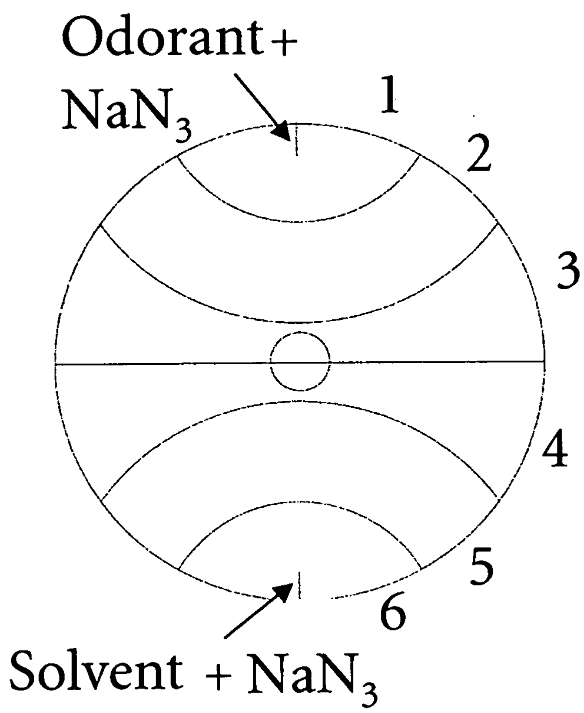


Figure 3B

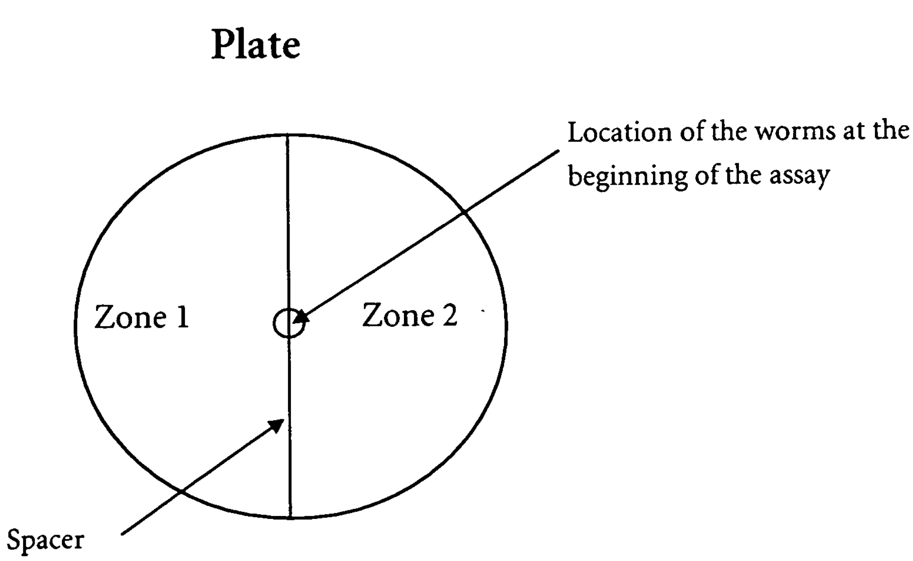

Figure 4A

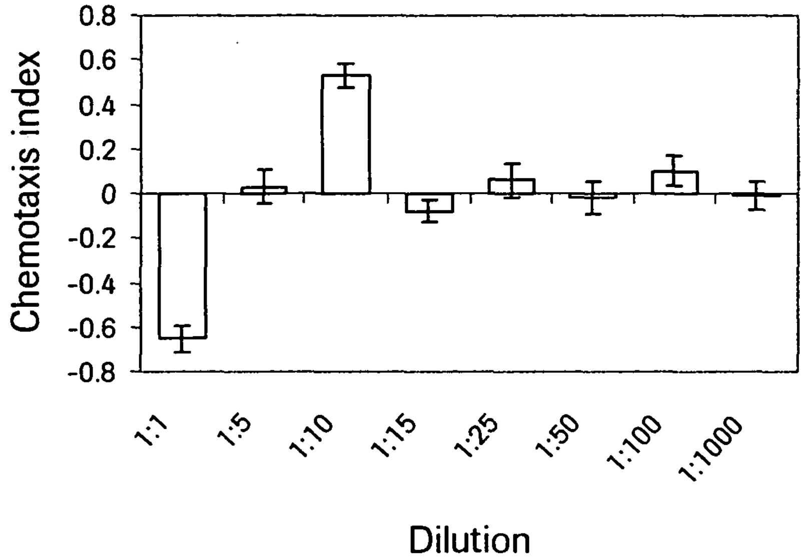


Figure 4B

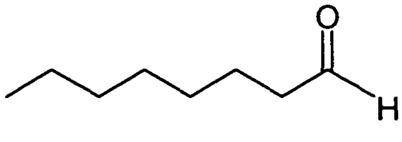

Octyl aldehyde

Figure 5A

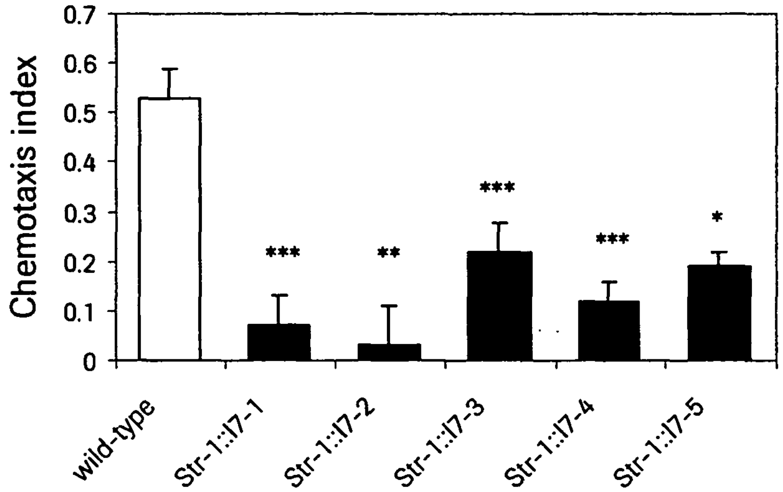


Figure 5B

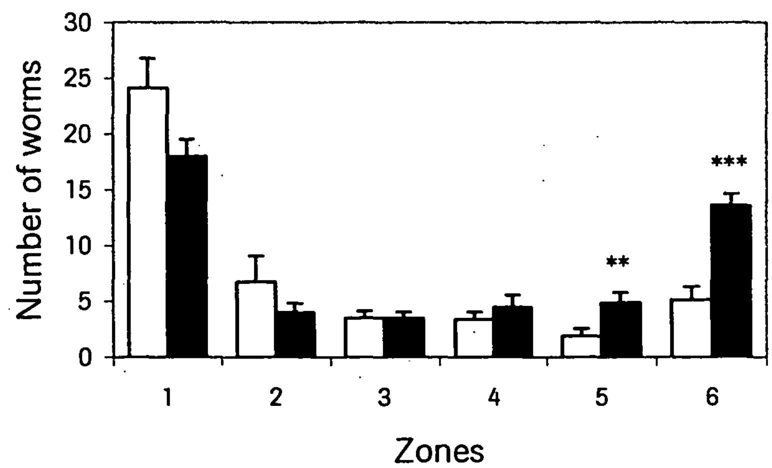

Figure 5C

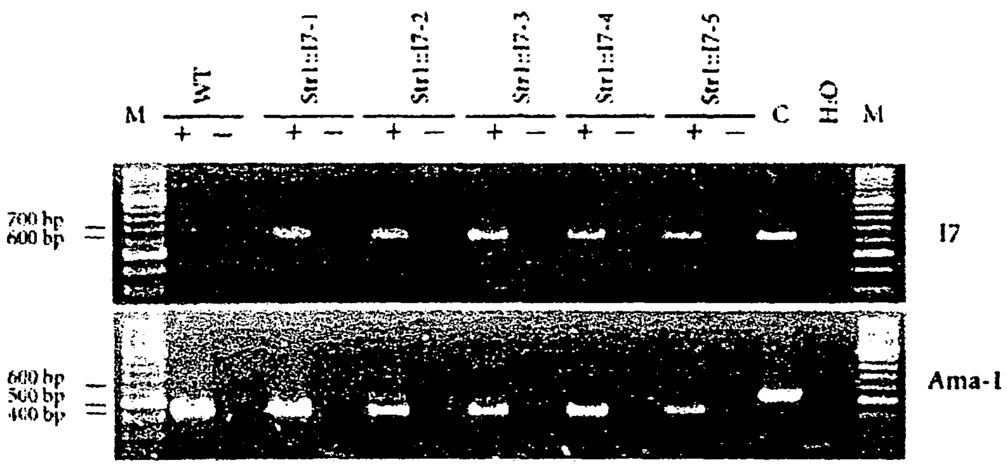
M
WT
Str1::I7-1
Str1::I7-2
Str1::I7-3
Str1::I7-4
Str1::I7-5
C
H2O
M
+ −
+ −
+ −
+ −
+ −
+ −
700 bp
600 bp
I7
600 bp
500 bp
400 bp
Ama-1

Figure 6A

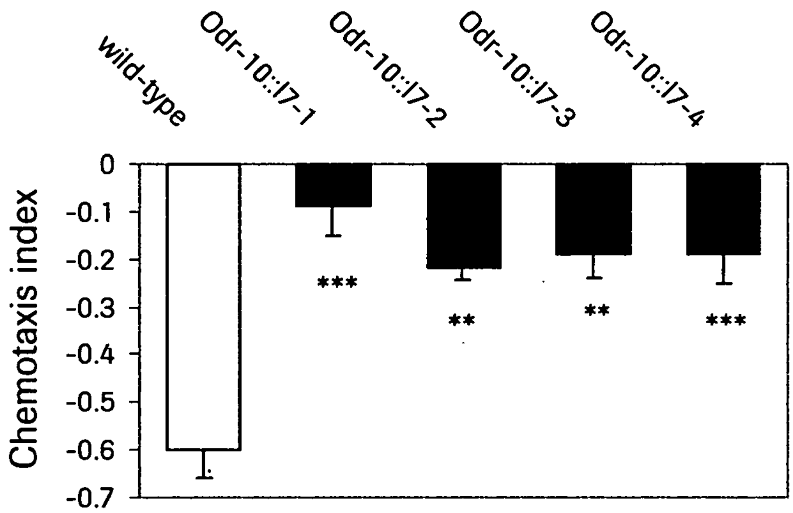


Figure 6B

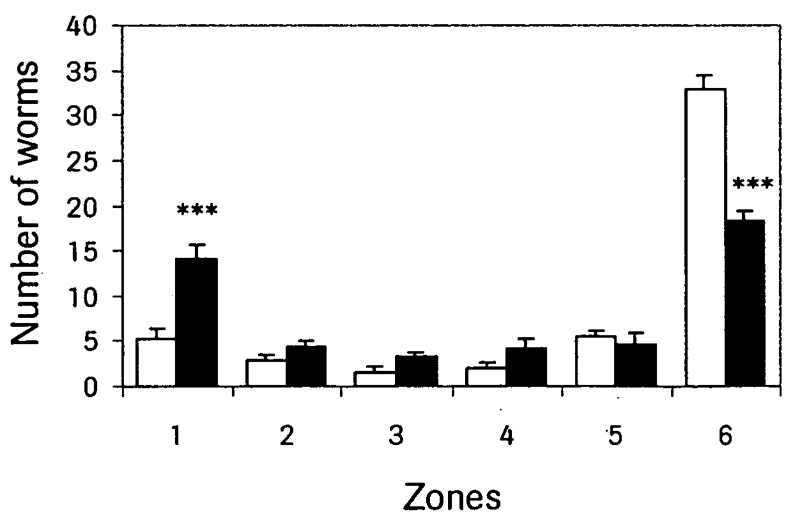

Figure 6C

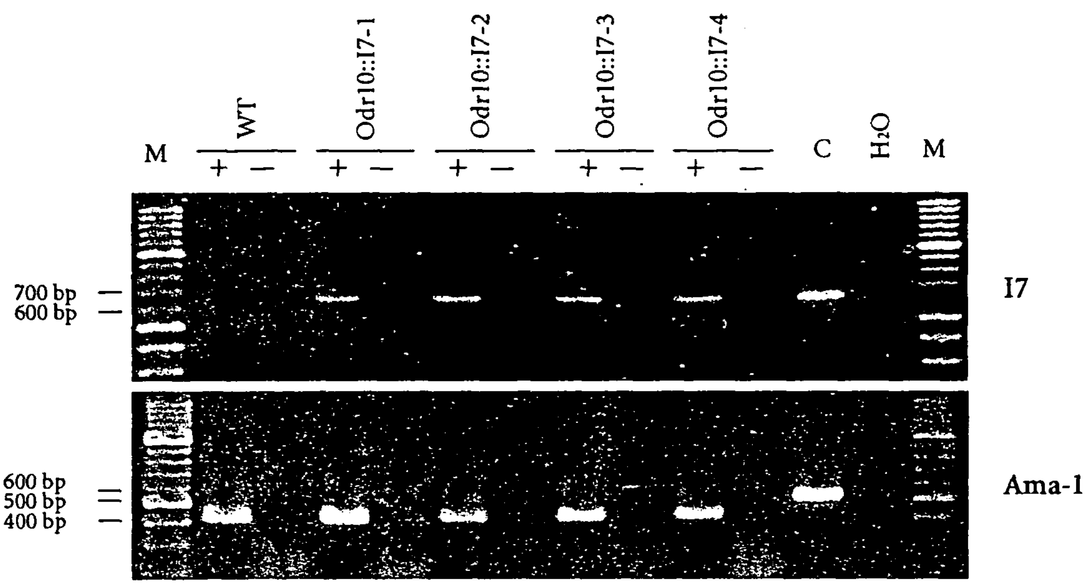
M
WT
+ −
Odr10::I7-1
+ −
Odr10::I7-2
+ −
Odr10::I7-3
+ −
Odr10::I7-4
+ −
C
H2O
M
700 bp
600 bp
I7
600 bp
500 bp
400 bp
Ama-1

Figure 7

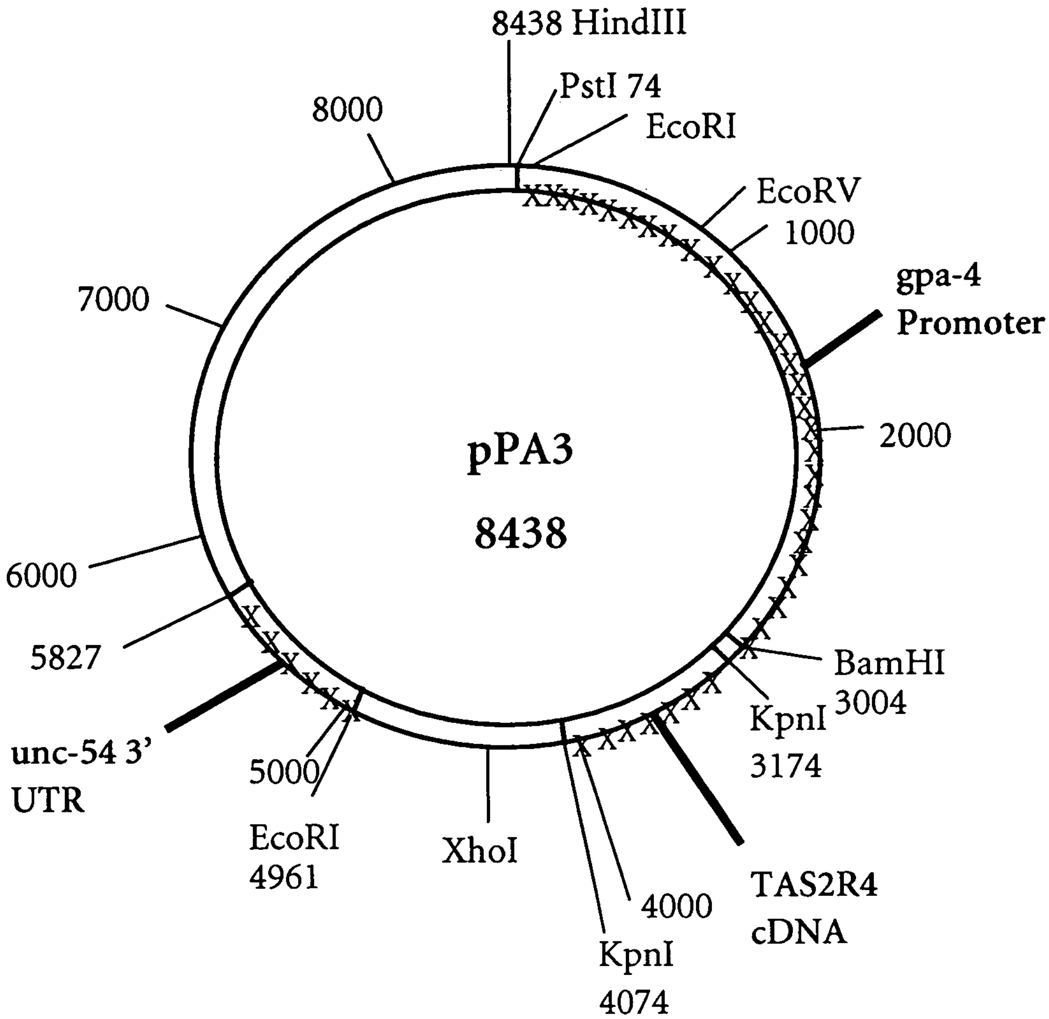
8438 HindIII
PstI 74
EcoRI
EcoRV
1000
gpa-4
Promoter
2000
BamHI
3004
KpnI
3174
TAS2R4
cDNA
4000
KpnI
4074
XhoI
EcoRI
4961
5000
unc-54 3'
UTR
5827
6000
7000
8000
pPA3
8438

Figure 8

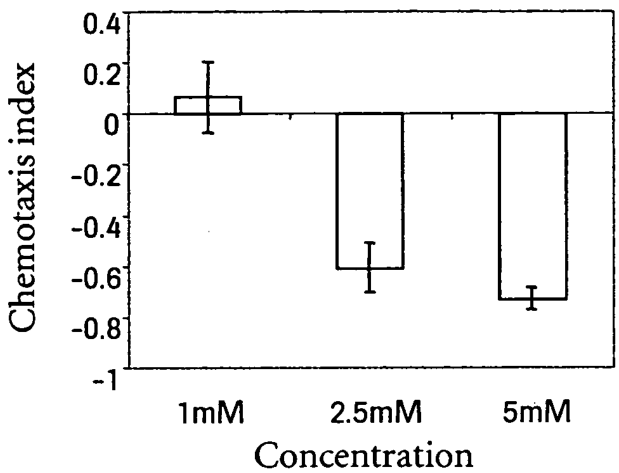


Figure 9

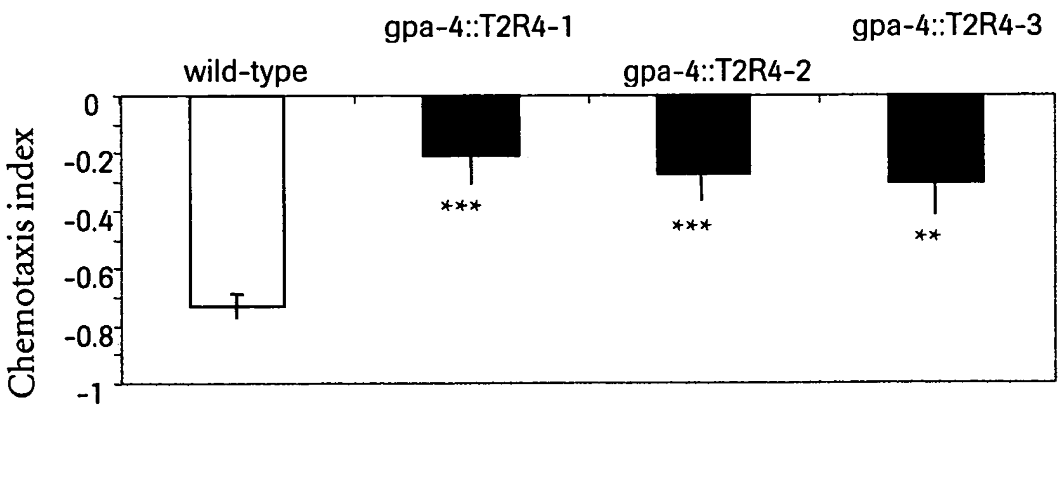

European Patent Office

# EUROPEAN SEARCH REPORT

Application Number
EP 03 01 7320

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 192 856 A (SMITHKLINE BEECHAM CORP) 3 April 2002 (2002-04-03)<br>* the whole document *<br>----- | 1-28 | C12N15/00<br>A01K67/033<br>C07K14/705<br>G01N33/50<br>A61K49/00 |
| X | TOBIN D, MADSEN D, KAHN-KIRBY A, PECKOL E, MOULDER G, BARSTEAD R, MARICQ A, BARGMANN C: "Combinatorial expression of TRPV channel proteins defines their sensory functions and subcellular localization in C. elegans neurons."<br>NEURON,<br>vol. 35, no. 2, 18 July 2002 (2002-07-18), pages 307-318, XP002263509<br>* page 313, column 1, paragraph 6 - page 315, column 2; figure 6 *<br>----- | 1,4,6-9, 11-13, 16-18, 22-26 | |
| X | TOBIN D. ET AL: "Roles of osm-9/capsaicin receptor family members in sensory behaviors"<br>WEST COAST WORM MEETING ABSTRACT,<br>23 June 2000 (2000-06-23), XP0002258309<br>INTERNET ARTICLE,<br>* abstract *<br>----- | 1,4,6-9, 11,12 | |
| X | WO 02/059152 A (HUDSPETH ALBERT JAMES ; HELLER STEFAN (US); FRIEDMAN JEFFREY M (US); L) 1 August 2002 (2002-08-01)<br>* claim 12; figure 15; example 4 *<br>-----<br>-/-- | 1-26 | |

| TECHNICAL FIELDS SEARCHED (Int.Cl.7) |
|---|
| C12N<br>A01K |

The present search report has been drawn up for all claims

| Place of search | Date of completion of the search | Examiner |
|---|---|---|
| The Hague | 2 December 2003 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 01 7320

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,X | NICOLETTA MILANI; ERNESTO GUARIN; EDUARD RENFER; PATRICK NEF*; PEDRO J. ANDRES-BARQUIN*CA: "Functional expression of a mammalian olfactory receptor in Caenorhabditis elegans" NEUROREPORT, vol. 13, no. 18, 20 December 2002 (2002-12-20), pages 2515-2520, XP0008025286 * the whole document * | 1-28 | |
| P,X | MILANI N; GUARIN E; RENFER E; NEF P; ANDRES-BARQUIN P J: "Expression of a mammalian olfactory G-protein-coupled receptor in C. elegans." MOLECULAR BIOLOGY OF THE CELL, vol. 13, November 2002 (2002-11), pages 123A-124A, XP0008025287 * the whole document * | 1-28 | |
| O,P,X | & 42nd Annual Meeting of the American Society for Cell Biology; San Francisco, CA, USA; December 14-18, 2002 * abstract * | 1-28 | TECHNICAL FIELDS SEARCHED (Int.Cl.7) |

The present search report has been drawn up for all claims

| Place of search | Date of completion of the search | Examiner |
|---|---|---|
| The Hague | 2 December 2003 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 03 01 7320

This annex lists the patent family membersrelating to the patent documents cited in the above-mentioned European search report.
The members are as contained in the European Patent Office EDP file on
The European Patent Office is in no way liable for these particulars which are merely given for the purpose of information.

02-12-2003

| Patent document cited in search report | | Publication date | Patent family member(s) | | Publication date |
|---|---|---|---|---|---|
| EP 1192856 | A | 03-04-2002 | EP | 1192856 A2 | 03-04-2002 |
| | | | US | 2002124275 A1 | 05-09-2002 |
| WO 02059152 | A | 01-08-2002 | US | 2003013650 A1 | 16-01-2003 |
| | | | WO | 02059152 A2 | 01-08-2002 |

EPO FORM P0459

For more details about this annex : see Official Journal of the European Patent Office, No. 12/82